# EUROPÄISCHE PATENTANMELDUNG

(11) **EP 3 501 607 A1**
(43) Veröffentlichungstag der Anmeldung: **26.06.2019**
(21) Anmeldenummer: 17210504.1
(22) Anmeldetag: 22.12.2017
(51) Int. Cl.: A61P 11/00, A61K 9/00, A61K 31/7088, C12N 15/113, A61P 29/00, A61P 11/06

(54) **ZUSAMMENSETZUNG ZUR BEHANDLUNG EINES AN EINER MIT CHRONISCHEN ENTZÜNDUNGEN EINHERGEHENDEN ATEMWEGSERKRANKUNG LEIDENDEN PATIENTEN SOWIE HERSTELLUNGSVERFAHREN UND VERWENDUNG DER ZUSAMMENSETZUNG**

(71) Anmelder: Sterna Biologicals GmbH & Co. KG, 35037 Marburg (DE)
(72) Erfinder: Renz, Jonas, 81675 München (DE)
(74) Vertreter: Patentanwälte Olbricht Buchhold Keulertz

(57) **Zusammenfassung**

Die Erfindung betrifft eine Zusammensetzung zur Behandlung eines Patienten, der an einer mit chronischen Entzündungen einhergehenden Atemwegserkrankung leidet, wobei die Zusammensetzung mindestens ein DNAzym umfasst, das die Expression von GATA-3 spezifisch herunterreguliert und/oder mindestens ein DNAzym umfasst, das die Expression von Tbet spezifisch herunterreguliert. Die Zusammensetzung ist dadurch gekennzeichnet, dass die Konzentration des DNAzyms in der Zusammensetzung niedriger als 75 mg/ml ist. Die Erfindung betrifft außerdem ein Verfahren zur Herstellung der erfindungsgemäßen Zusammensetzung sowie eine Verwendung der Zusammensetzung Arzneimittel zur Behandlung eines an einer mit chronischen Entzündungen einhergehenden Atemwegserkrankung leidenden Patienten.

SEQUENCE LISTING

<110> Sterna Biologicals GmbH & Co. KG

<120> Zusammensetzung zur Behandlung eines an einer mit chronischen
Entzündungen einhergehenden Atemwegserkrankungen leidenden Patienten sowie
Herstellungsverfahren und Verwendung der Zusammensetzung

<140> EP2017
<141> 2017-12-22

<160> 148

<170> BiSSAP 1.3.5

<210> 1
<211> 33
<212> DNA
<213> Homo sapiens

<400> 1
tcggtcagag gctagctaca acgatgcgtt gct                                    33

<210> 2
<211> 33
<212> DNA
<213> Homo sapiens

<400> 2
ggcgtacgag gctagctaca acgactgctc ggt                                    33

<210> 3
<211> 33
<212> DNA
<213> Homo sapiens

<400> 3
ggcggcgtag gctagctaca acgagacctg ctc                                    33

<210> 4
<211> 33
<212> DNA
<213> Homo sapiens

<400> 4
ctcgggtcag gctagctaca acgactgggt agc                                    33

<210> 5
<211> 33
<212> DNA
<213> Homo sapiens

<400> 5
tcctctgcag gctagctaca acgacggggt cct                                    33

<210> 6
<211> 33
<212> DNA
<213> Homo sapiens

<400> 6
actctgcaag gctagctaca acgatctgcg agc                                    33

<210> 7
<211> 33
<212> DNA
<213> Homo sapiens

<400> 7
gggcgacgag gctagctaca acgatctgca att                                    33

<210> 8
<211> 33
<212> DNA
<213> Homo sapiens

<400> 8
aaggggcgag gctagctaca acgagactct gca                                    33

<210> 9
<211> 33
<212> DNA
<213> Homo sapiens

<400> 9
aaaacgggag gctagctaca acgacaggtt gta                                    33

<210> 10
<211> 33
<212> DNA
<213> Homo sapiens

<400> 10
agaataaaag gctagctaca acgagggacc agg                                    33

<210> 11
<211> 33
<212> DNA
<213> Homo sapiens

<400> 11
atggcagaag gctagctaca acgaaaaacg gga                                    33

<210> 12

<211> 33
<212> DNA
<213> Homo sapiens

<400> 12
aactgggtag gctagctaca acgaggcaga ata                                    33

<210> 13
<211> 33
<212> DNA
<213> Homo sapiens

<400> 13
atccaaaaag gctagctaca acgatgggta tgg                                    33

<210> 14
<211> 33
<212> DNA
<213> Homo sapiens

<400> 14
aggggaagag gctagctaca acgaaaaaat cca                                    33

<210> 15
<211> 33
<212> DNA
<213> Homo sapiens

<400> 15
ttttaaaaag gctagctaca acgatatctt gga                                    33

<210> 16
<211> 33
<212> DNA
<213> Homo sapiens

<400> 16
gtggggggag gctagctaca acgagggaag gct                                    33

<210> 17
<211> 33
<212> DNA
<213> Homo sapiens

<400> 17
gttgaatgag gctagctaca acgattgctt tcg                                    33

<210> 18
<211> 33
<212> DNA
<213> Homo sapiens

<400> 18
gtcgttgaag gctagctaca acgagatttg ctt                                    33

<210> 19
<211> 33
<212> DNA
<213> Homo sapiens

<400> 19
ggcccggaag gctagctaca acgaccgcgc gcg                                    33

<210> 20
<211> 33
<212> DNA
<213> Homo sapiens

<400> 20
tcacctccag gctagctaca acgaggcctc ggc                                    33

<210> 21
<211> 33
<212> DNA
<213> Homo sapiens

<400> 21
ccgccgtcag gctagctaca acgactccat ggc                                    33

<210> 22
<211> 33
<212> DNA
<213> Homo sapiens

<400> 22
ggtggctcag gctagctaca acgaccagcg cgg                                    33

<210> 23
<211> 33
<212> DNA
<213> Homo sapiens

<400> 23
cgttgagcag gctagctaca acgaggcggg gtg                                    33

<210> 24
<211> 33
<212> DNA
<213> Homo sapiens

<400> 24

ccgcgtccag gctagctaca acgagtagga gtg 33

<210> 25
<211> 33
<212> DNA
<213> Homo sapiens

<400> 25
cagcgggtag gctagctaca acgatgcgcc gcg 33

<210> 26
<211> 33
<212> DNA
<213> Homo sapiens

<400> 26
gcacatccag gctagctaca acgactcctc cgg 33

<210> 27
<211> 33
<212> DNA
<213> Homo sapiens

<400> 27
aaaagcacag gctagctaca acgaccacct cct 33

<210> 28
<211> 33
<212> DNA
<213> Homo sapiens

<400> 28
taaaaagcag gctagctaca acgaatccac ctc 33

<210> 29
<211> 33
<212> DNA
<213> Homo sapiens

<400> 29
gaccgtcgag gctagctaca acgagttaaa aag 33

<210> 30
<211> 33
<212> DNA
<213> Homo sapiens

<400> 30
ttgccttgag gctagctaca acgacgtcga tgt 33

<210> 31
<211> 33
<212> DNA
<213> Homo sapiens

<400> 31
agggcgggag gctagctaca acgagtggtt gcc 33

<210> 32
<211> 33
<212> DNA
<213> Homo sapiens

<400> 32
tggccctgag gctagctaca acgacgagtt tcc 33

<210> 33
<211> 33
<212> DNA
<213> Homo sapiens

<400> 33
acctctgcag gctagctaca acgacgtggc cct 33

<210> 34
<211> 33
<212> DNA
<213> Homo sapiens

<400> 34
cggagggtag gctagctaca acgactctgc acc 33

<210> 35
<211> 33
<212> DNA
<213> Homo sapiens

<400> 35
ggcggcacag gctagctaca acgactggct ccc 33

<210> 36
<211> 33
<212> DNA
<213> Homo sapiens

<400> 36
cgggcggcag gctagctaca acgaacctgg ctc 33

<210> 37
<211> 33
<212> DNA

<213> Homo sapiens

<400> 37
agggatccag gctagctaca acgagaagca gag                                    33

<210> 38
<211> 33
<212> DNA
<213> Homo sapiens

<400> 38
gggtagggag gctagctaca acgaccatga agc                                    33

<210> 39
<211> 33
<212> DNA
<213> Homo sapiens

<400> 39
gggctgagag gctagctaca acgatccagg ggg                                    33

<210> 40
<211> 33
<212> DNA
<213> Homo sapiens

<400> 40
gtggatggag gctagctaca acgagtcttg gag                                    33

<210> 41
<211> 33
<212> DNA
<213> Homo sapiens

<400> 41
cgtggtggag gctagctaca acgaggacgt ctt                                    33

<210> 42
<211> 33
<212> DNA
<213> Homo sapiens

<400> 42
gggggtagag gctagctaca acgaggagag ggg                                    33

<210> 43
<211> 33
<212> DNA
<213> Homo sapiens

<400> 43
ggaggaggag gctagctaca acgagaggcc ggg                                    33

<210> 44
<211> 33
<212> DNA
<213> Homo sapiens

<400> 44
gccccccgag gctagctaca acgaaaggag gag                                    33

<210> 45
<211> 33
<212> DNA
<213> Homo sapiens

<400> 45
ccggggagag gctagctaca acgagtcctt cgg                                    33

<210> 46
<211> 33
<212> DNA
<213> Homo sapiens

<400> 46
ggacagcgag gctagctaca acgagggtcc ggg                                    33

<210> 47
<211> 33
<212> DNA
<213> Homo sapiens

<400> 47
tggggtggag gctagctaca acgaagcgat ggg                                    33

<210> 48
<211> 33
<212> DNA
<213> Homo sapiens

<400> 48
cttgaggcag gctagctaca acgatctttc tcg                                    33

<210> 49
<211> 33
<212> DNA
<213> Homo sapiens

<400> 49
cacctggtag gctagctaca acgattgagg cac                                    33

<210> 50
<211> 33
<212> DNA
<213> Homo sapiens

<400> 50
gcaggggcag gctagctaca acgactggta ctt                                    33

<210> 51
<211> 33
<212> DNA
<213> Homo sapiens

<400> 51
ccagcttcag gctagctaca acgagctgtc ggg                                    33

<210> 52
<211> 33
<212> DNA
<213> Homo sapiens

<400> 52
gtgggacgag gctagctaca acgatccagc ttc                                    33

<210> 53
<211> 33
<212> DNA
<213> Homo sapiens

<400> 53
ggagtgggag gctagctaca acgagactcc agc                                    33

<210> 54
<211> 33
<212> DNA
<213> Homo sapiens

<400> 54
atgctgccag gctagctaca acgagggagt ggg                                    33

<210> 55
<211> 33
<212> DNA
<213> Homo sapiens

<400> 55
gggcggtcag gctagctaca acgagctgcc acg                                    33

<210> 56
<211> 33

<212> DNA
<213> Homo sapiens

<400> 56
gaggctccag gctagctaca acgaccaggg cgg                                    33

<210> 57
<211> 33
<212> DNA
<213> Homo sapiens

<400> 57
gtgggtcgag gctagctaca acgagaggag gct                                    33

<210> 58
<211> 33
<212> DNA
<213> Homo sapiens

<400> 58
aggtggtgag gctagctaca acgaggggtg gtg                                    33

<210> 59
<211> 33
<212> DNA
<213> Homo sapiens

<400> 59
actcgggcag gctagctaca acgagtaggg cgg                                    33

<210> 60
<211> 33
<212> DNA
<213> Homo sapiens

<400> 60
ggagctgtag gctagctaca acgatcgggc acg                                    33

<210> 61
<211> 33
<212> DNA
<213> Homo sapiens

<400> 61
ggacttgcag gctagctaca acgaccgaag ccg                                    33

<210> 62
<211> 33
<212> DNA
<213> Homo sapiens

<400> 62
gggcctggag gctagctaca acgattgcat ccg                                    33

<210> 63
<211> 33
<212> DNA
<213> Homo sapiens

<400> 63
tgtgctggag gctagctaca acgacgggcc ttg                                    33

<210> 64
<211> 33
<212> DNA
<213> Homo sapiens

<400> 64
gttcacacag gctagctaca acgatccctg cct                                    33

<210> 65
<211> 33
<212> DNA
<213> Homo sapiens

<400> 65
cagttcacag gctagctaca acgaactccc tgc                                    33

<210> 66
<211> 33
<212> DNA
<213> Homo sapiens

<400> 66
cacagttcag gctagctaca acgaacactc cct                                    33

<210> 67
<211> 33
<212> DNA
<213> Homo sapiens

<400> 67
gttgccccag gctagctaca acgaagttca cac                                    33

<210> 68
<211> 33
<212> DNA
<213> Homo sapiens

<400> 68
tcgccgccag gctagctaca acgaagtggg gtc                                    33

<210> 69
<211> 33
<212> DNA
<213> Homo sapiens

<400> 69
cccgtgccag gctagctaca acgactcgcc gcc                                    33

<210> 70
<211> 33
<212> DNA
<213> Homo sapiens

<400> 70
ggcgttgcag gctagctaca acgaaggtag tgt                                    33

<210> 71
<211> 33
<212> DNA
<213> Homo sapiens

<400> 71
tggcttctag gctagctaca acgagccctc gtc                                    33

<210> 72
<211> 33
<212> DNA
<213> Homo sapiens

<400> 72
gggctctgag gctagctaca acgagcctgg ctt                                    33

<210> 73
<211> 33
<212> DNA
<213> Homo sapiens

<400> 73
gggaccccag gctagctaca acgacggagc ccg                                    33

<210> 74
<211> 33
<212> DNA
<213> Homo sapiens

<400> 74
ggtgggggag gctagctaca acgacccacc gga                                    33

<210> 75

<211> 33
<212> DNA
<213> Homo sapiens

<400> 75
ggcgggggag gctagctaca acgaccgagg gcc 33

<210> 76
<211> 33
<212> DNA
<213> Homo sapiens

<400> 76
gggctgggag gctagctaca acgagggcag gga 33

<210> 77
<211> 33
<212> DNA
<213> Homo sapiens

<400> 77
cgtcgaggag gctagctaca acgaccgccc ctc 33

<210> 78
<211> 33
<212> DNA
<213> Homo sapiens

<400> 78
gggctggcag gctagctaca acgacttccc gta 33

<210> 79
<211> 33
<212> DNA
<213> Homo sapiens

<400> 79
cgatgcccag gctagctaca acgaccgggg cgg 33

<210> 80
<211> 33
<212> DNA
<213> Homo sapiens

<400> 80
gctccacgag gctagctaca acgagcccat ccg 33

<210> 81
<211> 33
<212> DNA
<213> Homo sapiens

<400> 81
ccggctccag gctagctaca acgagatgcc cat                                    33

<210> 82
<211> 33
<212> DNA
<213> Homo sapiens

<400> 82
tctccgcaag gctagctaca acgaccggct cca                                    33

<210> 83
<211> 33
<212> DNA
<213> Homo sapiens

<400> 83
ccgtcagcag gctagctaca acgagtctcc gca                                    33

<210> 84
<211> 33
<212> DNA
<213> Homo sapiens

<400> 84
tccccggcag gctagctaca acgacggctc ggt                                    33

<210> 85
<211> 33
<212> DNA
<213> Homo sapiens

<400> 85
cccccgcgag gctagctaca acgagctcgt ccg                                    33

<210> 86
<211> 33
<212> DNA
<213> Homo sapiens

<400> 86
gtagggagag gctagctaca acgacccagg ctg                                    33

<210> 87
<211> 33
<212> DNA
<213> Homo sapiens

<400> 87

gggcgggcag gctagctaca acgacaaggc gcc                                    33

<210> 88
<211> 33
<212> DNA
<213> Homo sapiens

<400> 88
cgggaaggag gctagctaca acgatcgccc gcg                                    33

<210> 89
<211> 33
<212> DNA
<213> Homo sapiens

<400> 89
tagtcctcag gctagctaca acgagcggcc ccg                                    33

<210> 90
<211> 33
<212> DNA
<213> Homo sapiens

<400> 90
tccccgacag gctagctaca acgactccag tcc                                    33

<210> 91
<211> 33
<212> DNA
<213> Homo sapiens

<400> 91
tttccccgag gctagctaca acgaacctcc agt                                    33

<210> 92
<211> 33
<212> DNA
<213> Homo sapiens

<400> 92
tgagcgcgag gctagctaca acgacctcag ttt                                    33

<210> 93
<211> 33
<212> DNA
<213> Homo sapiens

<400> 93
ggaccacaag gctagctaca acgaaggtgg ttg                                    33

<210> 94
<211> 33
<212> DNA
<213> Homo sapiens

<400> 94
cttggaccag gctagctaca acgaaacagg tgg                                    33

<210> 95
<211> 33
<212> DNA
<213> Homo sapiens

<400> 95
aaacttggag gctagctaca acgacacaac agg                                    33

<210> 96
<211> 33
<212> DNA
<213> Homo sapiens

<400> 96
ctgattaaag gctagctaca acgattggac cac                                    33

<210> 97
<211> 33
<212> DNA
<213> Homo sapiens

<400> 97
tggtgctgag gctagctaca acgataaact tgg                                    33

<210> 98
<211> 33
<212> DNA
<213> Homo sapiens

<400> 98
tgatgatcag gctagctaca acgactctgt ctg                                    33

<210> 99
<211> 33
<212> DNA
<213> Homo sapiens

<400> 99
tggtgatgag gctagctaca acgacatctc tgt                                    33

<210> 100
<211> 33
<212> DNA

<213> Homo sapiens

<400> 100
gcttggtgag gctagctaca acgagatcat ctc 33

<210> 101
<211> 33
<212> DNA
<213> Homo sapiens

<400> 101
atgggaacag gctagctaca acgaccgccg tcc 33

<210> 102
<211> 33
<212> DNA
<213> Homo sapiens

<400> 102
gaatgggaag gctagctaca acgaatccgc cgt 33

<210> 103
<211> 33
<212> DNA
<213> Homo sapiens

<400> 103
tgacaggaag gctagctaca acgagggaac atc 33

<210> 104
<211> 33
<212> DNA
<213> Homo sapiens

<400> 104
agtaaatgag gctagctaca acgaaggaat ggg 33

<210> 105
<211> 33
<212> DNA
<213> Homo sapiens

<400> 105
cacagtaaag gctagctaca acgagacagg aat 33

<210> 106
<211> 33
<212> DNA
<213> Homo sapiens

<400> 106
gcccggccag gctagctaca acgaagtaaa tga                                    33

<210> 107
<211> 33
<212> DNA
<213> Homo sapiens

<400> 107
ccacaaacag gctagctaca acgacctgta gtg                                    33

<210> 108
<211> 33
<212> DNA
<213> Homo sapiens

<400> 108
gtccacaaag gctagctaca acgaatcctg tag                                    33

<210> 109
<211> 33
<212> DNA
<213> Homo sapiens

<400> 109
ccacgtccag gctagctaca acgaaaacat cct                                    33

<210> 110
<211> 33
<212> DNA
<213> Homo sapiens

<400> 110
ccaagaccag gctagctaca acgagtccac aaa                                    33

<210> 111
<211> 33
<212> DNA
<213> Homo sapiens

<400> 111
ccaccaagag gctagctaca acgacacgtc cac                                    33

<210> 112
<211> 33
<212> DNA
<213> Homo sapiens

<400> 112
gctggtccag gctagctaca acgacaagac cac                                    33

<210> 113
<211> 33
<212> DNA
<213> Homo sapiens

<400> 113
gctctggtag gctagctaca acgacgccag tgg 33

<210> 114
<211> 33
<212> DNA
<213> Homo sapiens

<400> 114
ctgcacccag gctagctaca acgattgccg ctc 33

<210> 115
<211> 33
<212> DNA
<213> Homo sapiens

<400> 115
cacactgcag gctagctaca acgaccactt gcc 33

<210> 116
<211> 33
<212> DNA
<213> Homo sapiens

<400> 116
ctttccacag gctagctaca acgatgcacc cac 33

<210> 117
<211> 33
<212> DNA
<213> Homo sapiens

<400> 117
gcctttccag gctagctaca acgaactgca ccc 33

<210> 118
<211> 33
<212> DNA
<213> Homo sapiens

<400> 118
ttcctggcag gctagctaca acgagctgcc ctc 33

<210> 119
<211> 33

<212> DNA
<213> Homo sapiens

<400> 119
gtggacgtag gctagctaca acgaaggcgg ttt 33

<210> 120
<211> 33
<212> DNA
<213> Homo sapiens

<400> 120
ccgggtggag gctagctaca acgagtacag gcg 33

<210> 121
<211> 33
<212> DNA
<213> Homo sapiens

<400> 121
cctggcgcag gctagctaca acgaccagtg cgc 33

<210> 122
<211> 33
<212> DNA
<213> Homo sapiens

<400> 122
caaatgaaag gctagctaca acgattcctg gcg 33

<210> 123
<211> 33
<212> DNA
<213> Homo sapiens

<400> 123
tttcccaaag gctagctaca acgagaaact tcc 33

<210> 124
<211> 33
<212> DNA
<213> Homo sapiens

<400> 124
attgttggag gctagctaca acgagccccc ttg 33

<210> 125
<211> 33
<212> DNA
<213> Homo sapiens

<400> 125
tgggtcacag gctagctaca acgatgttgg acg                                    33

<210> 126
<211> 33
<212> DNA
<213> Homo sapiens

<400> 126
tctgggtcag gctagctaca acgaattgtt gga                                    33

<210> 127
<211> 33
<212> DNA
<213> Homo sapiens

<400> 127
gcacaatcag gctagctaca acgactgggt cac                                    33

<210> 128
<211> 33
<212> DNA
<213> Homo sapiens

<400> 128
ggagcacaag gctagctaca acgacatctg ggt                                    33

<210> 129
<211> 33
<212> DNA
<213> Homo sapiens

<400> 129
actggagcag gctagctaca acgaaatcat ctg                                    33

<210> 130
<211> 33
<212> DNA
<213> Homo sapiens

<400> 130
atggagggag gctagctaca acgatggagc aca                                    33

<210> 131
<211> 33
<212> DNA
<213> Homo sapiens

<400> 131
tggtacttag gctagctaca acgaggaggg act                                    33

<210> 132
<211> 33
<212> DNA
<213> Homo sapiens

<400> 132
gggctggtag gctagctaca acgattatgg agg                                    33

<210> 133
<211> 33
<212> DNA
<213> Homo sapiens

<400> 133
tcaacgatag gctagctaca acgagcagcc ggg                                    33

<210> 134
<211> 33
<212> DNA
<213> Homo sapiens

<400> 134
cctcaacgag gctagctaca acgaatgcag ccg                                    33

<210> 135
<211> 33
<212> DNA
<213> Homo sapiens

<400> 135
tcacctcaag gctagctaca acgagatatg cag                                    33

<210> 136
<211> 33
<212> DNA
<213> Homo sapiens

<400> 136
cgtcgttcag gctagctaca acgactcaac gat                                    33

<210> 137
<211> 33
<212> DNA
<213> Homo sapiens

<400> 137
gtaaagatag gctagctaca acgagcgtgt tgg                                    33

<210> 138

<211> 33
<212> DNA
<213> Homo sapiens

<400> 138
aagtaaagag gctagctaca acgaatgcgt gtt                                    33

<210> 139
<211> 33
<212> DNA
<213> Homo sapiens

<400> 139
ggcaatgaag gctagctaca acgatgggtt tct                                    33

<210> 140
<211> 33
<212> DNA
<213> Homo sapiens

<400> 140
tcacggcaag gctagctaca acgagaactg ggt                                    33

<210> 141
<211> 33
<212> DNA
<213> Homo sapiens

<400> 141
aggcagtcag gctagctaca acgaggcaat gaa                                    33

<210> 142
<211> 33
<212> DNA
<213> Homo sapiens

<400> 142
atctcggcag gctagctaca acgatctggt agg                                    33

<210> 143
<211> 33
<212> DNA
<213> Homo sapiens

<400> 143
gctgagtaag gctagctaca acgactcggc att                                    33

<210> 144
<211> 33
<212> DNA
<213> Homo sapiens

<400> 144
tattatcaag gctagctaca acgatttcag ctg                                    33

<210> 145
<211> 33
<212> DNA
<213> Homo sapiens

<400> 145
gggttattag gctagctaca acgacaattt tca                                    33

<210> 146
<211> 33
<212> DNA
<213> Homo sapiens

<400> 146
aaggggttag gctagctaca acgatatcaa ttt                                    33

<210> 147
<211> 33
<212> DNA
<213> Homo sapiens

<400> 147
ctcccggaag gctagctaca acgacctttg gca                                    33

<210> 148
<211> 33
<212> DNA
<213> Homo sapiens

<400> 148
gtacatggag gctagctaca acgatcaaag ttc                                    33

## Beschreibung

Die Erfindung betrifft eine Zusammensetzung zur Behandlung eines an einer mit chronischen Entzündungen einhergehenden Atemwegserkrankung leidenden Patienten nach Anspruch 1, ein Verfahren zur Herstellung der Zusammensetzung nach Anspruch 10 sowie die Verwendung einer solchen Zusammensetzung nach Anspruch 12.

Falls nicht anders angegeben, werden die Begriffe "Expression" und "Genexpression" im Folgenden synonym verwendet.

Chronische Entzündungen sind in der Medizin von zunehmender Bedeutung. Entsprechend wichtig ist es, Therapien für Erkrankungen wie zum Beispiel Erkrankungen der Atemwege zu finden, die mit chronischen Entzündungen einhergehen. Solche Atemwegserkrankungen sind zum Beispiel allergische Soforttypreaktionen, Asthma oder chronisch-obstruktive Lungenerkrankungen (COPD).

An der Entstehung von chronischen Entzündungsreaktionen sowie von (oftmals auch entzündungsabhängigen) Autoimmunerkrankungen sind unter anderem zwei Transkriptionsfaktoren beteiligt: der Th1-zellspezifische Transkriptionsfaktor Tbet und der Th2-zellspezifische Transkriptionsfaktor GATA-3. Dabei induziert Tbet die spezifische Entwicklung von Th1-Zellen; GATA-3 induziert hingegen die spezifische Entwicklung von Th2-Zellen. Die Balance zwischen Th1- und Th2-Zellen wird also durch die Expression von GATA-3 bzw. durch die Inhibition von Tbet zugunsten der Th2-Zellen verschoben. Analog sorgen die Inhibition von GATA-3 bzw. die Expression von Tbet für ein steigendes Th1-Zellen-Niveau. Zu dem speziellen Gleichgewicht zwischen GATA-3 und Tbet bzw. zwischen Th1- und Th2-Zellen sind zahlreiche wissenschaftliche Publikationen erschienen.

Folglich sind in den letzten Jahren Versuche unternommen worden, die Transkriptionsfaktoren GATA-3 und Tbet spezifisch zu hemmen bzw. "auszuschalten". Eine Möglichkeit, das zu bewerkstelligen, stellt die Inhibition der Genexpression dieser beiden Transkriptionsfaktoren dar, wozu die moderne Gentechnologie diverse "Werkzeuge" zur Verfügung stellt. Eines dieser Werkzeuge sind DNAzyme.

DNAzyme repräsentieren eine verhältnismäßig neue Klasse von antisense-Molekülen. Als *antisense*-Moleküle werden Moleküle bezeichnet, die fähig sind, einzelsträngige Nukleinsäuren (i.d.R. mRNA) spezifisch und im Wesentlichen komplementär zu binden. Eine Besonderheit von DNAzymen ist dabei, dass sie neben dieser Bindefunktion auch eine katalytische Funktion aufweisen. Sie sind also in der Lage, spezifisch einzelsträngige Ziel-DNA oder -RNA zu spalten und somit abzubauen (Sel et al. 2008). Man spricht in diesem Fall auch von posttranskriptioneller Inhibition, weil die Inhibition der Genexpression auf mRNA-Ebene stattfindet, also noch bevor sich die Translation der mRNA zu einem Protein anschließen kann

Üblicherweise werden dazu DNAzyme des 10-23-Modells verwendet (Sontoro et al., 1997). Derartige DNAzyme besitzen eine katalytische Domäne von 15 Nukleotiden, welche von zwei Substratbindungsdomänen flankiert wird. Die katalytische Domäne kann dabei insbesondere die konservierte Sequenz *ggctagctacaacga* umfassen (SEQ ID No. 154). Die angegebene Sequenz *ggctagctacaacga* ist lediglich eine bevorzugte Ausführungsform. Dem Fachmann ist bekannt, dass DNAzyme des 10-23-Modells mit abgewandelter katalytischer Domäne eine vergleichbare biologische Aktivität besitzen können. Die Länge der Substratbindungsdomänen ist hingegen variabel, wobei sich auch zwei korrespondierende Substratbindungsdomänen voneinander unterscheiden können. In einer bevorzugten Ausführung, beträgt die Länge der Substratbindungsdomänen zwischen 6 und 14 Nukleotide, wobei eine Länge von 9 Nukleotiden besonders bevorzugt wird. Solche DNAzyme weisen eine spezifische Sequenz wie zum Beispiel *nnnnnnnnnggctagctacaacgannnnnnnnn* auf. In der Regel sind die Substratbindungsdomänen vollständig komplementär zu den Regionen, die die vorgesehene Spaltstelle der Ziel-mRNA flankieren - um die Ziel-RNA zu binden und zu spalten, muss das DNAzym jedoch nicht unbedingt vollständig komplementär sein. DNAzyme des 10-23 Typs spalten die Ziel-mRNA an Purin-Pyrimidin Abfolge-Sequenzen.

DNAzyme sind ebenso bekannt wie ihre Verwendung zur spezifischen Inhibition der Expression von GATA-3 bzw. Tbet. So wird zum Beispiel in der WO 2005/033314 A2 die Wirkweise verschiedener DNAzyme beschrieben. Der Offenbarungsgehalt der WO 2005/033314 wird als technologischer Hintergrund der vorliegenden Erfindung angesehen.

Probleme, die bei der Verwendung von DNAzymen als Wirkstoff auftreten können, sind oftmals auf die verhältnismäßig hohe Instabilität und die Sensibilität von Nukleinsäuren zurückzuführen. Sofern sie nicht in physiologisch günstigen Lösungen vorliegen, neigen Nukleinsäuren zur schnellen Degradierung, etwa durch enzymatischen Abbau oder physikalischen Stress - dies gilt insbesondere für einzelsträngige Nukleinsäuren, zu denen auch DNAzyme zählen.

Darüber hinaus schließt sich bei jeder Verwendung eines Wirkstoffs zu Therapiezwecken die Frage nach der Applizierbarkeit des Wirkstoffs an. So ist vor allem im Bereich der Atemwegserkrankungen, die mit Entzündungen einhergehen, eine örtlich gezielte Applikation nötig, möchte man den Entzündungsherd effizient bekämpfen bzw. die Zielzellen gezielt erreichen. Dementsprechend ist etwa im Falle von allergischen Soforttypreaktionen, Asthma, oder COPD ein Wirkstoff dann am effektivsten, wenn er über die Lunge aufgenommen werden kann.

Eine Möglichkeit, Atemwegserkrankungen gezielt zu bekämpfen, ist zum Beispiel die Inhalation. Über die Inhalation können Patienten Wirkstoffe in die Lunge bzw. über die Zielzellen in der Lunge aufnehmen, wodurch der Wirkstoff ortsspezifisch wirken oder rasch über die Alveolen in den Blutkreislauf aufgenommen werden kann. Diese Methode kann die Risiken für Nebenwirkungen im restlichen Organismus verringern.

Zusätzlich ist eine geringere Dosis notwendig, weil ein wesentlich größerer Dosisanteil den Wirkungsort erreicht als durch unspezifische Applikation.

In der Praxis übliche Behandlungsstrategien von Patienten mit Entzündungserkrankungen beschränken sich vor allem auf Wirkstoffe, die eine stark immunsuppressive Wirkung besitzen. Beispiele für solche Wirkstoffe sind Corticosteroide, Mesalazin (5-Aminosalicylsäure), Ciclosporin, Tacrolimus oder TNHα-Antikörper. Dabei eignet sich keiner der vorbenannten Therapiewege dazu, gezielt eingesetzt zu werden. Vielmehr sind die eingesetzten Substanzen für ihre stark immunsuppressive Wirkung bekannt. Darüber hinaus stehen die Stoffe mit häufig auftretenden Nebenwirkungen in Verbindung, wie zum Beispiel Immunsuppression, Hepatotoxizität, Myokardhypertrophie gastrointestinale Beschwerden, Müdigkeit und Schwindel, Diarrhö, Erbrechen, Nephrotoxizität, Neurotoxizität, Tremor, Hypertonie, Insomnie, Depressionen, Krämpfe, Maskierung von Infektionen, Hyperkaliämie, Hyperglykämie, erhöhtes Tumorrisiko - um nur einige zu nennen.

Der Erfindung liegt daher die Aufgabe zugrunde, eine DNAzym-haltige Zusammensetzung zur Behandlung eines an einer mit chronischen Entzündungen einhergehenden Atemwegserkrankung leidenden Patienten bereitzustellen, die sich zur gezielten Applikation eignet. Insbesondere soll die Zusammensetzung über die Lunge des zu behandelnden Patienten aufgenommen werden können, weshalb sie leicht zu zerstäuben sein sollte. Darüber hinaus soll der Wirkstoff das Risiko von Nebenwirkungen geringhalten.

Diese Aufgabe wird erfindungsgemäß durch eine Zusammensetzung zur Behandlung eines Patienten gelöst, der an einer mit chronischen Entzündungen einhergehenden Atemwegserkrankung leidet, wobei die Zusammensetzung mindestens ein DNAzym umfasst, das die Expression von GATA-3 spezifisch herunterreguliert und/oder mindestens ein DNAzym umfasst, das die Expression von Tbet spezifisch herunterreguliert. Dabei ist die Zusammensetzung dadurch gekennzeichnet, dass das DNAzym in der Zusammensetzung in einer Konzentration vorliegt, die niedriger als 75 mg/ml ist. Es kann insbesondere vorgesehen sein, dass die Zusammensetzung als Aerosol applizierbar ist, das über die Lunge aufgenommen wird. Vorzugsweise kann die Zusammensetzung eine Flüssigkeit oder eine Lösung sein, alternativ ein Pulver.

Dem Fachmann ist bekannt, dass unter einer Herunterregulation einer Expression sowohl eine teilweise als auch eine vollständige Inhibition der Expression verstanden werden kann. In jedem Fall wird die Expression im Vergleich zum natürlichen Expressionsniveau signifikant verringert. Vorliegend binden und spalten die spezifischen DNAzyme *in vivo* die mRNA der Transkriptionsfaktoren GATA-3 und/oder Tbet, deren Proteine zentrale Schlüsselmoleküle für die Entstehung von Th1- bzw. Th2-abhängigen chronischen Entzündungserkrankungen darstellen. Durch diese Spaltung der mRNA können die mRNAs nicht mehr in funktionale Proteine translatiert werden. Folglich wird das GATA-3- bzw. Tbet-Proteinniveau signifikant minimiert.

Die erfindungsgemäße Zusammensetzung mit einer DNAzym-Konzentration, die niedriger ist als 75 mg/ml, lässt sich dabei ideal zur Behandlung von Patienten einsetzen, die an einer mit chronischen Entzündung einhergehenden Atemwegserkrankung leiden. Das liegt vor allem daran, dass Zusammensetzungen mit DNAzym-Konzentrationen, die 75 mg/ml übersteigen, mit einem Wirkstoffverlust verbunden sind. Grund hierfür ist, dass sich derart hochkonzentrierte Zusammensetzungen aufgrund ihrer erhöhten Viskosität nicht mehr problemlos zerstäuben lassen (etwa über ein Inhalationsgerät). In der Medizin übliche Handinhalatoren arbeiten in der Regel mit einem Zerstäubungsdruck von 40 bar bis 45 bar. Für ein derartiges Inhalationsgerät ist die Oberflächenspannung einer Lösung mit einer DNAzym-Konzentration von über 75 mg/ml zu hoch, um noch zerstäubbar zu sein. Als Folge zeigen bereits Zusammensetzungen mit einer DNAzym-Konzentration von 75 mg/ml einen Wirkstoffverlust von etwa 6 %, wenn sie mittels eines Inhalationsgeräts appliziert werden. Demgegenüber besitzen insbesondere Zusammensetzungen mit einer DNAzym-Konzentration von 20 mg/ml bis 50 mg/ml einen optimalen Wirkungseffekt bei der Behandlung mit einem Inhalator, weil sich Zusammensetzungen mit derartigen Konzentrationen einfach zerstäuben lassen. Entsprechend wird erfindungsgemäß eine Zusammensetzung bevorzugt, die eine DNAzym-Konzentration von 20 mg/ml bis 50 mg/ml aufweist.

Die erfindungsgemäße Zusammensetzung kann dabei insbesondere als Inhalationslösung vorliegen, die als Aerosol in die Lunge eines Patienten appliziert werden kann, was vor allem bei der Behandlung von Atemwegserkrankungen von Vorteil ist, die durch allergische Entzündungsreaktionen ausgelöst werden. Bei solchen Atemwegserkrankungen können die Wirkstoffe (DNAzyme) durch eine erfindungsgemäße Zusammensetzung in Aerosolform im Wesentlichen an Ort und Stelle der Entzündung appliziert werden. Als Folge ergibt sich daraus eine höhere Effizienz bei der Behandlung entsprechender Patienten. Dazu ist insbesondere vorgesehen, dass die Zusammensetzung eine Flüssigkeit oder eine Lösung ist. Alternativ kann die Zusammensetzung ein Pulver sein.

Das erfindungsgemäße Merkmal, wonach die DNAzym-Konzentration niedriger ist als 75 mg/ml, ist dabei insbesondere bei der Verwendung von DNAzymen von großem Vorteil. Für wässrige DNA-Lösungen sind üblicherweise Konzentrationen möglich, die 75 mg/ml weit übersteigen, ohne dass dabei die Viskosität der Lösung signifikant erhöht wird. So können gerade kleinere DNA-Moleküle (< 1.000 bp), zu denen auch DNAzyme zählen, in Konzentrationen von über 100 mg/ml vorliegen, ohne dass die Lösungen dabei besonders viskös werden.

Bei DNAzymen zeigt sich jedoch der überraschende Effekt, dass DNAzym-haltige Lösungen bereits bei Konzentrationen von mindestens 75 mg/ml überproportional stark viskos werden. Bei Konzentrationen von über 75 mg/ml fallen sie sogar aus. Grund hierfür ist vor allem die dreidimensionale Struktur der DNAzyme, die durch die Ausbildung eines "loops" oder einer "Schlaufe" gekennzeichnet ist. Aufgrund dieser besonderen Struktur können DNAzyme - befördert durch ihre Einzelsträngigkeit - eine Art Polymerstruktur ausbilden und leicht miteinander verklumpen, was wiederum eine insgesamt schlechte Löslichkeit hoher DNAzym-Mengen zur Folge hat. Für die ortsspezifische Behandlung von Patienten, die an einer mit chronischen Entzündung einhergehenden Atemwegserkrankung leiden, ist die erfindungsgemäße Zusammensetzung also von großem Vorteil, weil sie aufgrund einer DNAzym-Konzentration von unter 75 mg/ml nicht zu viskös ist und sich leicht daher als Aerosol verabreichen lässt.

Es ist insbesondere vorgesehen, dass die Zusammensetzung eine DNAzym-Konzentration von 20 mg/ml bis 50 mg/ml aufweist. Mit einer derartigen Zusammensetzung wird gewährleistet, dass auch bei der Verwendung von Handinhalatoren oder vergleichbaren Inhalationsgeräten ausreichende Mengen an Wirkstoff durch den Patienten aufgenommen werden können. Bei Zusammensetzungen mit DNAzym-Konzentrationen von unter 20 mg/ml besteht nämlich das Risiko, dass die Wirkstoffmengen, die durch eine einzelne Auslösung oder Betätigung eines Inhalators abgegeben werden, nicht ausreichen, um den erwünschten Behandlungserfolg herbeizuführen - es werden pro Auslösung des Inhalators schlicht zu wenige Wirkstoffe abgegeben. Folglich müssen Patienten das Inhalationsgerät mehrmals betätigen, was zum einen eine mögliche Belastung für die zu behandelnden Patienten darstellt und zum anderen für einen höheren Verschleiß der Inhalatoren sorgt. Durch eine hohe Wirkstoffkonzentration sind außerdem mehrere Applikationen pro Inhalator möglich.

Die erfindungsgemäße Zusammensetzung gewährleistet also vor allem in dieser bevorzugten Ausführungsform einen idealen Kompromiss: Einerseits ist die DNAzym-Konzentration mit weniger als 50 mg/ml noch niedrig genug, um noch durch ein Inhalationsgerät zerstäubt werden zu können, andererseits ist die DNAzym-Konzentration mit mehr als 20 mg/ml hoch genug, dass zum Beispiel bei der Verwendung von Handinhalatoren bereits nach wenigen Betätigungen ausreichende Wirkstoffmengen abgegeben werden können.

Gemäß einer vorteilhaften Weiterentwicklung kann außerdem vorgesehen sein, dass die Zusammensetzung eine Viskosität aufweist, die niedriger ist als 3,5 mPa·s. Eine derartige Viskosität hat zur Folge, dass sich die Zusammensetzung problemlos und ohne signifikanten Wirkstoffverlust als Aerosol applizieren lässt, etwa als Inhalationslösung eines Inhalators. Eine höhere Viskosität hat zur Folge, dass der Druck, der zum Zerstäuben der Zusammensetzung aufgewendet werden muss, zu hoch ist, um noch durch ein gewöhnliches Inhalationsgerät ausgeübt werden zu können. Somit kann eine Zusammensetzung mit einer Viskosität von über 3,5 mPa·s dazu führen, dass die Zerstäuber der Inhalatoren, die üblicherweise zur Behandlung entsprechender Patienten eingesetzt werden, bereits nach kurzer Zeit verstopfen oder verschmieren. Es ist also vorteilhaft, dass die Viskosität der Zusammensetzung in einem Bereich liegt, der eine möglichst hohe Wirkstoffkonzentration (DNAzyme) bei verhältnismäßig geringer Viskosität gewährleistet.

Beispielhaft aber nicht abschließend werden als DNAzyme die DNAzyme der früheren Anmeldung DE 103 46 487 genannt und in diese Anmeldung aufgenommen. Diese DNAzyme sind gegen die mRNA der Proteine GATA-3 und T-bet gerichtet und zur Herstellung eines Mittels gegen entzündliche Erkrankungen offenbart.

Beispielsweise werden folgende DNAzyme verwendet (jedes einzeln oder in Kombination mit den anderen):
Name der DNAzyme gegen GATA-3 mRNA-Sequenz:
   SEQ ID No: 1 hgd1 5'-TCGGTCAGAggctagctacaacgaTGCGTTGCT-3'
   SEQ ID No: 2 hgd2 5'-GGCGTACGAggctagctacaacgaCTGCTCGGT-3'
   SEQ ID No: 3 hgd3 5'-GGCGGCGTAggctagctacaacgaGACCTGCTC-3'
   SEQ ID No: 4 hgd4 5'-CTCGGGTCAggctagctacaacgaCTGGGTAGC-3'
   SEQ ID No: 5 hgd5 5'-TCCTCTGCAggctagctacaacgaCGGGGTCCT-3'
   SEQ ID No: 6 hgd6 5'-ACTCTGCAAggctagctacaacgaTCTGCGAGC-3'
   SEQ ID No: 7 hgd7 5'-GGGCGACGAggctagctacaacgaTCTGCAATT-3'
   SEQ ID No: 8 hgd8 5'-AAGGGGCGAggctagctacaacgaGACTCTGCA-3'
   SEQ ID No: 9 hgd9 5'-AAAACGGGAggctagctacaacgaCAGGTTGTA-3'
   SEQ ID No: 10 hgd10 5'-AGAATAAAAggctagctacaacgaGGGACCAGG-3'
   SEQ ID No: 11 hgd11 5'-ATGGCAGAAggctagctacaacgaAAAACGGGA-3'
   SEQ ID No: 12 hgd12 5'-AACTGGGTAggctagctacaacgaGGCAGAATA-3'
   SEQ ID No: 13 hgd13 5'-ATCCAAAAAggctagctacaacgaTGGGTATGG-3'
   SEQ ID No: 14 hgd14 5'-AGGGGAAGAggctagctacaacgaAAAAATCCA-3'
   SEQ ID No: 15 hgd15 5'-TTTTAAAAAggctagctacaacgaTATCTTGGA-3'
   SEQ ID No: 16 hgd16 5'-GTGGGGGGAggctagctacaacgaGGGAAGGCT-3'
   SEQ ID No: 17 hgd17 5'-GTTGAATGAggctagctacaacgaTTGCTTTCG-3'
   SEQ ID No: 18 hgd18 5'-GTCGTTGAAggctagctacaacgaGATTTGCTT-3'
   SEQ ID No: 19 hgd19 5'-GGCCCGGAAggctagctacaacgaCCGCGCGCG-3'
   SEQ ID No: 20 hgd20 5'-TCACCTCCAggctagctacaacgaGGCCTCGGC-3'
   SEQ ID No: 21 hgd21 5'-CCGCCGTCAggctagctacaacgaCTCCATGGC-3'
   SEQ ID No: 22 hgd22 5'-GGTGGCTCAggctagctacaacgaCCAGCGCGG-3'
   SEQ ID No: 23 hgd23 5'-CGTTGAGCAggctagctacaacgaGGCGGGGTG-3'
   SEQ ID No: 24 hgd24 5'-CCGCGTCCAggctagctacaacgaGTAGGAGTG-3'
   SEQ ID No: 25 hgd25 5'-CAGCGGGTAggctagctacaacgaTGCGCCGCG-3'
   SEQ ID No: 26 hgd26 5'-GCACATCCAggctagctacaacgaCTCCTCCGG-3'
   SEQ ID No: 27 hgd27 5'-AAAAGCACAggctagctacaacgaCCACCTCCT-3'
   SEQ ID No: 28 hgd28 5'-TAAAAAGCAggctagctacaacgaATCCACCTC-3'
   SEQ ID No:29 hgd29 5'-GACCGTCGAggctagctacaacgaGTTAAAAAG-3'
   SEQ ID No: 30 hgd30 5'-TTGCCTTGAggctagctacaacgaCGTCGATGT-3'
   SEQ ID No: 31 hgd31 5'-AGGGCGGGAggctagctacaacgaGTGGTTGCC-3'
   SEQ ID No: 32 hgd32 5'-TGGCCCTGAggctagctacaacgaCGAGTTTCC-3'
   SEQ ID No: 33 hgd33 5'-ACCTCTGCAggctagctacaacgaCGTGGCCCT-3'
   SEQ ID No: 34 hgd34 5'-CGGAGGGTAggctagctacaacgaCTCTGCACC-3'
   SEQ ID No: 35 hgd35 5'-GGCGGCACAggctagctacaacgaCTGGCTCCC-3'
   SEQ ID No: 36 hgd36 5'-CGGGCGGCAggctagctacaacgaACCTGGCTC-3'
   SEQ ID No: 37 hgd37 5'-AGGGATCCAggctagctacaacgaGAAGCAGAG-3'
   SEQ ID No: 38 hgd38 5'-GGGTAGGGAggctagctacaacgaCCATGAAGC-3'
   SEQ ID No: 39 hgd39 5'-GGGCTGAGAggctagctacaacgaTCCAGGGGG-3'
   SEQ ID No: 40 hgd40 5'-GTGGATGGAggctagctacaacgaGTCTTGGAG-3'
   SEQ ID No: 41 hgd41 5'-CGTGGTGGAggctagctacaacgaGGACGTCTT-3'
   SEQ ID No: 42 hgd42 5'-GGGGGTAGAggctagctacaacgaGGAGAGGGG-3'
   SEQ ID No: 43 hgd43 5'-GGAGGAGGAggctagctacaacgaGAGGCCGGG-3'
   SEQ ID No: 44 hgd44 5'-GCCCCCCGAggctagctacaacgaAAGGAGGAG-3'
   SEQ ID No: 45 hgd45 5'-CCGGGGAGAggctagctacaacgaGTCCTTCGG-3'
   SEQ ID No: 46 hgd46 5'-GGACAGCGAggctagctacaacgaGGGTCCGGG-3'
   SEQ ID No: 47 hgd47 5'-TGGGGTGGAggctagctacaacgaAGCGATGGG-3'
   SEQ ID No: 48 hgd48 5'-CTTGAGGCAggctagctacaacgaTCTTTCTCG-3'
   SEQ ID No: 49 hgd49 5'-CACCTGGTAggctagctacaacgaTTGAGGCAC-3'
   SEQ ID No: 50 hgd50 5'-GCAGGGGCAggctagctacaacgaCTGGTACTT-3'
   SEQ ID No: 51 hgd51 5'-CCAGCTTCAggctagctacaacgaGCTGTCGGG-3'
   SEQ ID No: 52 hgd52 5'-GTGGGACGAggctagctacaacgaTCCAGCTTC-3'
   SEQ ID No: 53 hgd53 5'-GGAGTGGGAggctagctacaacgaGACTCCAGC-3'
   SEQ ID No: 54 hgd54 5'-ATGCTGCCAggctagctacaacgaGGGAGTGGG-3'
   SEQ ID No: 55 hgd55 5'-GGGCGGTCAggctagctacaacgaGCTGCCACG-3'
   SEQ ID No: 56 hgd56 5'-GAGGCTCCAggctagctacaacgaCCAGGGCGG-3'
   SEQ ID No: 57 hgd57 5'-GTGGGTCGAggctagctacaacgaGAGGAGGCT-3'
   SEQ ID No: 58 hgd58 5'-AGGTGGTGAggctagctacaacgaGGGGTGGTG-3'
   SEQ ID No: 59 hgd59 5'-ACTCGGGCAggctagctacaacgaGTAGGGCGG-3'
   SEQ ID No: 60 hgd60 5'-GGAGCTGTAggctagctacaacgaTCGGGCACG-3'
   SEQ ID No: 61 hgd61 5'-GGACTTGCAggctagctacaacgaCCGAAGCCG-3'
   SEQ ID No: 62 hgd62 5'-GGGCCTGGAggctagctacaacgaTTGCATCCG-3'
   SEQ ID No: 63 hgd63 5'-TGTGCTGGAggctagctacaacgaCGGGCCTTG-3'
   SEQ ID No: 64 hgd64 5'-GTTCACACAggctagctacaacgaTCCCTGCCT-3'
   SEQ ID No: 65 hgd65 5'-CAGTTCACAggctagctacaacgaACTCCCTGC-3'
   SEQ ID No: 66 hgd66 5'-CACAGTTCAggctagctacaacgaACACTCCCT-3'
   SEQ ID No: 67 hgd67 5'-GTTGCCCCAggctagctacaacgaAGTTCACAC-3'
   SEQ ID No: 68 hgd68 5'-TCGCCGCCAggctagctacaacgaAGTGGGGTC-3'
   SEQ ID No: 69 hgd69 5'-CCCGTGCCAggctagctacaacgaCTCGCCGCC-3'
   SEQ ID No: 70 hgd70 5'-GGCGTTGCAggctagctacaacgaAGGTAGTGT-3'
Name der DNAzyme gegen T-bet mRNASequenz:
   SEQ ID No: 71 td1 5'-TGGCTTCTAggctagctacaacgaGCCCTCGTC-3'
   SEQ ID No: 72 td2 5'-GGGCTCTGAggctagctacaacgaGCCTGGCTT-3'
   SEQ ID No: 73 td3 5'-GGGACCCCAggctagctacaacgaCGGAGCCCG-3'
   SEQ ID No: 74 td4 5'-GGTGGGGGAggctagctacaacgaCCCACCGGA-3'
   SEQ ID No: 75 td5 5'-GGCGGGGGAggctagctacaacgaCCGAGGGCC-3'
   SEQ ID No: 76 td6 5'-GGGCTGGGAggctagctacaacgaGGGCAGGGA-3'
   SEQ ID No: 77 td7 5'-CGTCGAGGAggctagctacaacgaCCGCCCCTC-3'
   SEQ ID No: 78 td8 5'-GGGCTGGCAggctagctacaacgaCTTCCCGTA-3'
   SEQ ID No: 79 td9 5'-CGATGCCCAggctagctacaacgaCCGGGGCGG-3'
   SEQ ID No: 80 td10 5'-GCTCCACGAggctagctacaacgaGCCCATCCG-3'
   SEQ ID No: 81 td11 5'-CCGGCTCCAggctagctacaacgaGATGCCCAT-3'
   SEQ ID No: 82 td12 5'-TCTCCGCAAggctagctacaacgaCCGGCTCCA-3'
   SEQ ID No: 83 td13 5'-CCGTCAGCAggctagctacaacgaGTCTCCGCA-3'
   SEQ ID No: 84 td14 5'-TCCCCGGCAggctagctacaacgaCGGCTCGGT-3'
   SEQ ID No: 85 td15 5'-CCCCCGCGAggctagctacaacgaGCTCGTCCG-3'
   SEQ ID No: 86 td16 5'-GTAGGGAGAggctagctacaacgaCCCAGGCTG-3'
   SEQ ID No: 87 td17 5'-GGGCGGGCAggctagctacaacgaCAAGGCGCC-3'
   SEQ ID No: 88 td18 5'-CGGGAAGGAggctagctacaacgaTCGCCCGCG-3'
   SEQ ID No: 89 td19 5'-TAGTCCTCAggctagctacaacgaGCGGCCCCG-3'
   SEQ ID No: 90 td20 5'-TCCCCGACAggctagctacaacgaCTCCAGTCC-3'
   SEQ ID No: 91 td21 5'-TTTCCCCGAggctagctacaacgaACCTCCAGT-3'
   SEQ ID No: 92 td22 5'-TGAGCGCGAggctagctacaacgaCCTCAGTTT-3'
   SEQ ID No: 93 td23 5'-GGACCACAAggctagctacaacgaAGGTGGTTG-3'
   SEQ ID No: 94 td24 5'-CTTGGACCAggctagctacaacgaAACAGGTGG-3'
   SEQ ID No: 95 td25 5'-AAACTTGGAggctagctacaacgaCACAACAGG-3'
   SEQ ID No: 96 td26 5'-CTGATTAAAggctagctacaacgaTTGGACCAC-3'
   SEQ ID No: 97 td27 5'-TGGTGCTGAggctagctacaacgaTAAACTTGG-3'
   SEQ ID No: 98 td28 5'-TGATGATCAggctagctacaacgaCTCTGTCTG-3'
   SEQ ID No: 99 td29 5'-TGGTGATGAggctagctacaacgaCATCTCTGT-3'
   SEQ ID No: 100 td30 5'-GCTTGGTGAggctagctacaacgaGATCATCTC-3'
   SEQ ID No: 101 td31 5'-ATGGGAACAggctagctacaacgaCCGCCGTCC-3'
   SEQ ID No: 102 td32 5'-GAATGGGAAggctagctacaacgaATCCGCCGT-3'
   SEQ ID No: 103 td33 5'-TGACAGGAAggctagctacaacgaGGGAACATC-3'
   SEQ ID No: 104 td34 5'-AGTAAATGAggctagctacaacgaAGGAATGGG-3'
   SEQ ID No: 105 td35 5'-CACAGTAAAggctagctacaacgaGACAGGAAT-3'
   SEQ ID No: 106 td36 5'-GCCCGGCCAggctagctacaacgaAGTAAATGA-3'
   SEQ ID No: 107 td37 5'-CCACAAACAggctagctacaacgaCCTGTAGTG-3'
   SEQ ID No: 108 td38 5'-GTCCACAAAggctagctacaacgaATCCTGTAG-3'
   SEQ ID No: 109 td39 5'-CCACGTCCAggctagctacaacgaAAACATCCT-3'
   SEQ ID No: 110 td40 5'-CCAAGACCAggctagctacaacgaGTCCACAAA-3'
   SEQ ID No: 111 td41 5'-CCACCAAGAggctagctacaacgaCACGTCCAC-3'
   SEQ ID No: 112 td42 5'-GCTGGTCCAggctagctacaacgaCAAGACCAC-3'
   SEQ ID No: 113 td43 5'-GCTCTGGTAggctagctacaacgaCGCCAGTGG-3'
   SEQ ID No: 114 td44 5'-CTGCACCCAggctagctacaacgaTTGCCGCTC-3'
   SEQ ID No: 115 td45 5'-CACACTGCAggctagctacaacgaCCACTTGCC-3'
   SEQ ID No: 116 td46 5'-CTTTCCACAggctagctacaacgaTGCACCCAC-3'
   SEQ ID No: 117 td47 5'-GCCTTTCCAggctagctacaacgaACTGCACCC-3'
   SEQ ID No: 118 td48 5'-TTCCTGGCAggctagctacaacgaGCTGCCCTC-3'
   SEQ ID No: 119 td49 5'-GTGGACGTAggctagctacaacgaAGGCGGTTT-3'
   SEQ ID No: 120 td50 5'-CCGGGTGGAggctagctacaacgaGTACAGGCG-3'
   SEQ ID No: 121 td51 5'-CCTGGCGCAggctagctacaacgaCCAGTGCGC-3'
   SEQ ID No: 122 td52 5'-CAAATGAAAggctagctacaacgaTTCCTGGCG-3'
   SEQ ID No: 123 td53 5'-TTTCCCAAAggctagctacaacgaGAAACTTCC-3'
   SEQ ID No: 124 td54 5'-ATTGTTGGAggctagctacaacgaGCCCCCTTG-3'
   SEQ ID No: 125 td55 5'-TGGGTCACAggctagctacaacgaTGTTGGACG-3'
   SEQ ID No: 126 td56 5'-TCTGGGTCAggctagctacaacgaATTGTTGGA-3
   SEQ ID No: 127 td57 5'-GCACAATCAggctagctacaacgaCTGGGTCAC-3'
   SEQ ID No: 128 td58 5'-GGAGCACAAggctagctacaacgaCATCTGGGT-3'
   SEQ ID No: 129 td59 5'-ACTGGAGCAggctagctacaacgaAATCATCTG-3'
   SEQ ID No: 130 td60 5'-ATGGAGGGAggctagctacaacgaTGGAGCACA-3'
   SEQ ID No: 131 td61 5'-TGGTACTTAggctagctacaacgaGGAGGGACT-3'
   SEQ ID No: 132 td62 5'-GGGCTGGTAggctagctacaacgaTTATGGAGG-3'
   SEQ ID No: 133 td63 5'-TCAACGATAggctagctacaacgaGCAGCCGGG-3'
   SEQ ID No: 134 td64 5'-CCTCAACGAggctagctacaacgaATGCAGCCG-3'
   SEQ ID No: 135 td65 5'-TCACCTCAAggctagctacaacgaGATATGCAG-3'
   SEQ ID No: 136 td66 5'-CGTCGTTCAggctagctacaacgaCTCAACGAT-3'
   SEQ ID No: 137 td67 5'-GTAAAGATAggctagctacaacgaGCGTGTTGG-3'
   SEQ ID No: 138 td68 5'-AAGTAAAGAggctagctacaacgaATGCGTGTT-3'
   SEQ ID No: 139 td69 5'-GGCAATGAAggctagctacaacgaTGGGTTTCT-3'
   SEQ ID No: 140 td70 5'-TCACGGCAAggctagctacaacgaGAACTGGGT-3'
   SEQ ID No: 141 td71 5'-AGGCAGTCAggctagctacaacgaGGCAATGAA-3'
   SEQ ID No: 142 td72 5'-ATCTCGGCAggctagctacaacgaTCTGGTAGG-3'
   SEQ ID No: 143 td73 5'-GCTGAGTAAggctagctacaacgaCTCGGCATT-3'
   SEQ ID No: 144 td74 5'-TATTATCAAggctagctacaacgaTTTCAGCTG-3'
   SEQ ID No: 145 td75 5'-GGGTTATTAggctagctacaacgaCAATTTTCA-3'
   SEQ ID No: 146 td76 5'-AAGGGGTTAggctagctacaacgaTATCAATTT-3'
   SEQ ID No: 147 td77 5'-CTCCCGGAAggctagctacaacgaCCTTTGGCA-3'
   SEQ ID No: 148 td78 5'-GTACATGGAggctagctacaacgaTCAAAGTTC-3'
Entsprechend sieht eine vorteilhafte Ausführungsform vor, dass das DNAzym ausgewählt ist aus einer Gruppe umfassend die DNAzyme hdg1 bis hdg70 (vgl .auch Tabelle 2) und/oder aus einer Gruppe umfassend die DNAzyme td1 bis td78 (vgl. Tabelle 2).

DNAzyme mit einer dieser Sequenzen erweisen sich als besonders effizient zur spezifischen *in vivo*-Inhibition der Expressionen der entzündungsfördernden Transkriptionsfaktoren GATA-3 bzw. Tbet. Generell werden dabei die Sequenzen bevorzugt, deren Substratbindungsdomänen vollständig komplementär zu den flankierenden Regionen der Spaltstelle einer bestimmten Ziel-mRNA sind. Ein DNAzym muss allerdings nicht vollständig komplementär sein, um eine Ziel-mRNA zu binden und spalten - es reichen gegebenenfalls kleinere komplementäre Sequenzabschnitte innerhalb der insgesamt homologen Substratbindungsdomäne.

Als besonders bevorzugte Variante ist eine Zusammensetzung vorgesehen, die zur Herunterregulation der Expression des Transkriptionsfaktors GATA-3 das DNAzym hdg40 GTGGATGGAggctagctacaacgaGTCTTGGAG (SEQ ID No. 40) aufweist.

Diese Sequenz zeigt eine besonders hohe Enzymaktivität und spaltet GATA-3-mRNA mit einer hohen Spezifität sowie mit hoher Effizienz. Folglich eignet sich ein DNAzym mit der Sequenz hgd40 (SEQ ID No. 40) hervorragend zur gezielten und effektiven Behandlung von Atemwegserkrankungen, die Th2-abhängig sind, die also mit einem erhöhten GATA-3-Spiegel korrespondieren.

Gemäß einer vorteilhaften Weiterbildung kann die Zusammensetzung mindestens einen Nuklease-Inhibitor aufweisen, wobei der mindestens eine Nuklease-Inhibitor insbesondere Desoxyribonukleasen spezifisch inaktiviert, also ein DNase Inhibitor ist.

Durch einen derartigen Nuklease-Inhibitor wird das mindestens eine DNAzym vor dem enzymatischen Abbau geschützt, der durch DNasen verursacht werden kann.

Es ist außerdem von Vorteil, wenn die Zusammensetzung mindestens ein Salz und/oder mindestens ein Kation umfasst.

Eine derartige Zusammensetzung eignet sich auf vorteilhafte Art und Weise zur Anwendung bei der Therapie von Patienten, die an einer mit chronischen Entzündungen einhergehenden Atemwegserkrankung leiden, weil die Zusammensetzung dadurch ein physiologisch günstiges Milieu aufweist und somit für Patienten leicht verträglich ist. Dabei ist insbesondere eine Phosphatgepufferte Salzlösung (PBS) vorgesehen. Es können aber auch andere gepufferte Lösungen vorgesehen sein, in denen Nukleinsäuren in physiologisch günstigem Milieu gelöst vorliegen können, wie zum Beispiel TE-Puffer. Entsprechend ist der anorganische und/oder organische Zusatz bevorzugterweise ausgewählt aus der Gruppe umfassend die Stoffe Natriumchlorid (NaCl), Kaliumchlorid (KCl), Dinatriumhydrogenphosphat (Na₂HPO₄), Dinatriumhydrogenphosphat Dihydrat, Kaliumdihydrogenphosphat (KH₂PO₄), TRIS und EDTA. Das Kation kann dabei ausgewählt sein aus der Gruppe umfassend Na, Mg, K, Li, Ca, Fe, Cu und Ag. Des Weiteren kann auch ein organisches Kation vorgesehen sein, wie zum Beispiel Mg(N(SO₂CF3)₂)₂ oder Mg(OSO₂CF₃)₂. Ferner kann ein bivalentes Kation vorgesehen sein.

Eine derartig gepufferte Zusammensetzung eignet sich besonders zum Schutz der DNAzyme, weil das DNAzym durch den anorganischen und/oder organischen Zusatz stabilisiert und vor enzymatischem Abbau geschützt wird. Darüber hinaus wird durch das Salz eine gute Aufnahme in die Zielzellen ermöglicht. Die bivalenten Kationen können als Co-Faktoren der DNAzyme die DNAzym-Aktivität steigern, da die DNAzyme eine von bivalenten Kationen (vorzugsweise Mg2+) abhängige katalytische Domäne aufweisen. Somit wirken bivalente Kationen als Enhancer oder Verstärker.

Nach einer vorteilhaften Weiterentwicklung kann die Zusammensetzung mindestens einen anorganischen und/oder organischen Zusatz und/oder einen Lösungsvermittler und/oder einen Konservierungsstoff umfassen. Der Lösungsvermittler dient dabei vor allem der Komplexbildung. Darüber hinaus verbessert er die Lösemitteleigenschaften der Zusammensetzung. Als Lösungsvermittler können bevorzugterweise Glycerolderivate und/oder Polyethylenglycole oder auch Lecithine vorgesehen sein.

Der Konservierungsstoff kann zum Beispiel Paraben sein. Ferner können Zusätze vorgesehen sein, die zum Beispiel eine Veränderung der Oberflächenspannung der Zusammensetzung oder eine Senkung der Viskosität der Zusammensetzung bewirken.

Ein zusätzlicher Aspekt dieser Erfindung ist das Verfahren zur Herstellung der erfindungsgemäßen Zusammensetzung, wobei vorgesehen ist, dass das Verfahren zumindest die folgenden Schritte umfasst:
a) Ansetzen einer DNAzym-haltigen Lösung, wobei die DNAzym-Konzentration in der wässrigen Lösung 80 mg/ml nicht übersteigt;
b) Filtration der Lösung aus Schritt a), bis die Lösung eine DNAzym-Konzentration von unter 75 mg/ml aufweist;

Der Filtrationsschritt ist von Vorteil, weil dadurch unerwünschte (unsterile) Stoffe sowie ungelöste Wirkstoffpartikel (also ungelöste DNAzyme) entfernt werden. Die Zusammensetzung muss zur Behandlung eines an einer mit chronischen Entzündungen einhergehenden Atemwegserkrankung leidenden Patienten steril sein. Andere Sterilisationsverfahren (Strahlung, Erhitzen, Autoklavieren etc.) sind hier nicht anwendbar, weil dadurch auch die DNAzyme zerstört würden. Aus diesem Grund ist vorgesehen, dass die Lösung mittels Filtration sterilisiert wird. Dabei wird bevorzugterweise ein Filter verwendet, der PES-Membran mit einem Porendurchmesser von 0,20 bis 0,25 µm aufweist. Besonders bevorzugt ist eine PES-Membran, mit einem Porendurchmesser von 0,22 µm.

Nach einer weiteren Ausgestaltung ist vorgesehen, dass das Verfahren mindestens einen weiteren Verfahrensschritt umfasst, namentlich ausgewählt aus einem der folgenden Schritte:
- Hinzufügen eines Nuklease-Inhibitors zur Lösung aus Schritt b);
- Hinzufügen des mindestens einen Salzes und/oder des mindestens einen Kations zur Lösung aus Schritt b);
- Hinzufügen des anorganischen und/oder organischen Zusatzes zur Lösung aus Schritt b);
- Hinzufügen des Lösungsvermittlers und/oder des Konservierungsstoffs zur Lösung aus Schritt b).

Ein derartiges Verfahren gewähreistet auf einfache Art und Weise die Herstellung einer Zusammensetzung, die zur Behandlung eines Patienten eingesetzt werden kann, der an einer mit chronischen Entzündungen einhergehenden Atemwegserkrankung leidet. Durch die Auswahl der Schritte lässt sich das Verfahren individuell an spezifische Bedürfnisse anpassen.

Einen weiteren Aspekt der Erfindung stellt die Verwendung der vorbenannten Zusammensetzung als Arzneimittel zur Behandlung eines an einer mit chronischen Entzündungen einhergehenden Atemwegserkrankung leidenden Patienten dar. Dazu kann die Zusammensetzung insbesondere als Inhalationslösung vorliegen. Denkbar sind aber auch andere Formen, wie etwa ein Pulver zu Anwendung in Pulverinhalatoren.

In einer bevorzugten Ausführungsform ist vorgesehen, dass die Zusammensetzung in Form eines Aerosols appliziert werden kann. Eine derartig applizierte Zusammensetzung kann ortsspezifisch in der Lunge eines an einer mit chronischen Entzündungen einhergehenden Atemwegserkrankung leidenden Patienten wirken. Dadurch kann die Effektivität des Arzneimittels erhöht werden. Gleichzeitig wird das Risiko von Nebenwirkungen minimiert.

Weitere Merkmale, Einzelheiten und Vorteile der Erfindung ergeben sich aus dem Wortlaut der Ansprüche sowie aus der folgenden Beschreibung von Ausführungsbeispielen.

Es zeigen:
- Tabelle 1: eine Liste der GATA-3-spezifischen DNAzyme;
- Tabelle 2: eine Liste der Tbet-spezifischen DNAzyme;
- Tabelle 3: ein Beispiel einer erfindungsgemäßen Zusammensetzung;
- Tabelle 4: mögliche zusätzliche Komponenten der Zusammensetzung aus Tabelle 3;
- Figur 1: grafische Darstellung der Viskosität einer DNAzym-haltigen Zusammensetzung in Abhängigkeit ihrer Konzentration;
- Figur 2: Viskositätsmessergebnisse einer DNAzym-haltigen Zusammensetzung in Abhängigkeit ihrer Konzentration;
- Figur 3: Messergebnisse eines FATs von Zusammensetzungen mit unterschiedlichen DNAzym-Konzentrationen;
- Figur 4: Messergebnisse eines FATs der erfindungsgemäßen Zusammensetzung mit DNAzym-Konzentrationen von 20 mg/ml bzw. 50 mg/ml.

### Ausführungsbeispiele

**Tabelle 1 - GATA-3-spezifische DNAzyme**

| **SEQ ID** | **Name** | **Sequenz** |
|---|---|---|
| SEQ ID No: 1 | hgd1 | 5'-TCGGTCAGAggctagctacaacgaTGCGTTGCT-3' |
| SEQ ID No: 2 | hgd2 | 5'-GGCGTACGAggctagctacaacgaCTGCTCGGT-3' |
| SEQ ID No: 3 | hgd3 | 5'-GGCGGCGTAggctagctacaacgaGACCTGCTC-3' |
| SEQ ID No: 4 | hgd4 | 5'-CTCGGGTCAggctagctacaacgaCTGGGTAGC-3' |
| SEQ ID No: 5 | hgd5 | 5'-TCCTCTGCAggctagctacaacgaCGGGGTCCT-3' |
| SEQ ID No: 6 | hgd6 | 5'-ACTCTGCAAggctagctacaacgaTCTGCGAGC-3' |
| SEQ ID No: 7 | hgd7 | 5'-GGGCGACGAggctagctacaacgaTCTGCAATT-3' |
| SEQ ID No: 8 | hgd8 | 5'-AAGGGGCGAggctagctacaacgaGACTCTGCA-3' |
| SEQ ID No: 9 | hgd9 | 5'-AAAACGGGAggctagctacaacgaCAGGTTGTA-3' |
| SEQ ID No: 10 | hgd10 | 5'-AGAATAAAAggctagctacaacgaGGGACCAGG-3' |
| SEQ ID No: 11 | hgd11 | 5'-ATGGCAGAAggctagctacaacgaAAAACGGGA-3' |
| SEQ ID No: 12 | hgd12 | 5'-AACTGGGTAggctagctacaacgaGGCAGAATA-3' |
| SEQ ID No: 13 | hgd13 | 5'-ATCCAAAAAggctagctacaacgaTGGGTATGG-3' |
| SEQ ID No: 14 | hgd14 | 5'-AGGGGAAGAggctagctacaacgaAAAAATCCA-3' |
| SEQ ID No: 15 | hgd15 | 5'-TTTTAAAAAggctagctacaacgaTATCTTGGA-3' |
| SEQ ID No: 16 | hgd16 | 5'-GTGGGGGGAggctagctacaacgaGGGAAGGCT-3' |
| SEQ ID No: 17 | hgd17 | 5'-GTTGAATGAggctagctacaacgaTTGCTTTCG-3' |
| SEQ ID No: 18 | hgd18 | 5'-GTCGTTGAAggctagctacaacgaGATTTGCTT-3' |
| SEQ ID No: 19 | hgd19 | 5'-GGCCCGGAAggctagctacaacgaCCGCGCGCG-3' |
| SEQ ID No: 20 | hgd20 | 5'-TCACCTCCAggctagctacaacgaGGCCTCGGC-3' |
| SEQ ID No: 21 | hgd21 | 5'-CCGCCGTCAggctagctacaacgaCTCCATGGC-3' |
| SEQ ID No: 22 | hgd22 | 5'-GGTGGCTCAggctagctacaacgaCCAGCGCGG-3' |
| SEQ ID No: 23 | hgd23 | 5'-CGTTGAGCAggctagctacaacgaGGCGGGGTG-3' |
| SEQ ID No: 24 | hgd24 | 5'-CCGCGTCCAggctagctacaacgaGTAGGAGTG-3' |
| SEQ ID No: 25 | hgd25 | 5'-CAGCGGGTAggctagctacaacgaTGCGCCGCG-3' |
| SEQ ID No: 26 | hgd26 | 5'-GCACATCCAggctagctacaacgaCTCCTCCGG-3' |
| SEQ ID No: 27 | hgd27 | 5'-AAAAGCACAggctagctacaacgaCCACCTCCT-3' |
| SEQ ID No: 28 | hgd28 | 5'-TAAAAAGCAggctagctacaacgaATCCACCTC-3' |
| SEQ ID No: 29 | hgd29 | 5'-GACCGTCGAggctagctacaacgaGTTAAAAAG-3' |
| SEQ ID No: 30 | hgd30 | 5'-TTGCCTTGAggctagctacaacgaCGTCGATGT-3' |
| SEQ ID No: 31 | hgd31 | 5'-AGGGCGGGAggctagctacaacgaGTGGTTGCC-3' |
| SEQ ID No: 32 | hgd32 | 5'-TGGCCCTGAggctagctacaacgaCGAGTTTCC-3' |
| SEQ ID No: 33 | hgd33 | 5'-ACCTCTGCAggctagctacaacgaCGTGGCCCT-3' |
| SEQ ID No: 34 | hgd34 | 5'-CGGAGGGTAggctagctacaacgaCTCTGCACC-3' |
| SEQ ID No: 35 | hgd35 | 5'-GGCGGCACAggctagctacaacgaCTGGCTCCC-3' |
| SEQ ID No: 36 | hgd36 | 5'-CGGGCGGCAggctagctacaacgaACCTGGCTC-3' |
| SEQ ID No: 37 | hgd37 | 5'-AGGGATCCAggctagctacaacgaGAAGCAGAG-3' |
| SEQ ID No: 38 | hgd38 | 5'-GGGTAGGGAggctagctacaacgaCCATGAAGC-3' |
| SEQ ID No: 39 | hgd39 | 5'-GGGCTGAGAggctagctacaacgaTCCAGGGGG-3' |
| SEQ ID No: 40 | hgd40 | 5'-GTGGATGGAggctagctacaacgaGTCTTGGAG-3' |
| SEQ ID No: 41 | hgd41 | 5'-CGTGGTGGAggctagctacaacgaGGACGTCTT-3' |
| SEQ ID No: 42 | hgd42 | 5'-GGGGGTAGAggctagctacaacgaGGAGAGGGG-3' |
| SEQ ID No: 43 | hgd43 | 5'-GGAGGAGGAggctagctacaacgaGAGGCCGGG-3' |
| SEQ ID No: 44 | hgd44 | 5'-GCCCCCCGAggctagctacaacgaAAGGAGGAG-3' |
| SEQ ID No: 45 | hgd45 | 5'-CCGGGGAGAggctagctacaacgaGTCCTTCGG-3' |
| SEQ ID No: 46 | hgd46 | 5'-GGACAGCGAggctagctacaacgaGGGTCCGGG-3' |
| SEQ ID No: 47 | hgd47 | 5'-TGGGGTGGAggctagctacaacgaAGCGATGGG-3' |
| SEQ ID No: 48 | hgd48 | 5'-CTTGAGGCAggctagctacaacgaTCTTTCTCG-3' |
| SEQ ID No: 49 | hgd49 | 5'-CACCTGGTAggctagctacaacgaTTGAGGCAC-3' |
| SEQ ID No: 50 | hgd50 | 5'-GCAGGGGCAggctagctacaacgaCTGGTACTT-3' |
| SEQ ID No: 51 | hgd51 | 5'-CCAGCTTCAggctagctacaacgaGCTGTCGGG-3' |
| SEQ ID No: 52 | hgd52 | 5'-GTGGGACGAggctagctacaacgaTCCAGCTTC-3' |
| SEQ ID No: 53 | hgd53 | 5'-GGAGTGGGAggctagctacaacgaGACTCCAGC-3' |
| SEQ ID No: 54 | hgd54 | 5'-ATGCTGCCAggctagctacaacgaGGGAGTGGG-3' |
| SEQ ID No: 55 | hgd55 | 5'-GGGCGGTCAggctagctacaacgaGCTGCCACG-3' |
| SEQ ID No: 56 | hgd56 | 5'-GAGGCTCCAggctagctacaacgaCCAGGGCGG-3' |
| SEQ ID No: 57 | hgd57 | 5'-GTGGGTCGAggctagctacaacgaGAGGAGGCT-3' |
| SEQ ID No: 58 | hgd58 | 5'-AGGTGGTGAggctagctacaacgaGGGGTGGTG-3' |
| SEQ ID No: 59 | hgd59 | 5'-ACTCGGGCAggctagctacaacgaGTAGGGCGG-3' |
| SEQ ID No: 60 | hgd60 | 5'-GGAGCTGTAggctagctacaacgaTCGGGCACG-3' |
| SEQ ID No: 61 | hgd61 | 5'-GGACTTGCAggctagctacaacgaCCGAAGCCG-3' |
| SEQ ID No: 62 | hgd62 | 5'-GGGCCTGGAggctagctacaacgaTTGCATCCG-3' |
| SEQ ID No: 63 | hgd63 | 5'-TGTGCTGGAggctagctacaacgaCGGGCCTTG-3' |
| SEQ ID No: 64 | hgd64 | 5'-GTTCACACAggctagctacaacgaTCCCTGCCT-3' |
| SEQ ID No: 65 | hgd65 | 5'-CAGTTCACAggctagctacaacgaACTCCCTGC-3' |
| SEQ ID No: 66 | hgd66 | 5'-CACAGTTCAggctagctacaacgaACACTCCCT-3' |
| SEQ ID No: 67 | hgd67 | 5'-GTTGCCCCAggctagctacaacgaAGTTCACAC-3' |
| SEQ ID No: 68 | hgd68 | 5'-TCGCCGCCAggctagctacaacgaAGTGGGGTC-3' |
| SEQ ID No: 69 | hgd69 | 5'-CCCGTGCCAggctagctacaacgaCTCGCCGCC-3' |
| SEQ ID No: 70 | hgd70 | 5'-GGCGTTGCAggctagctacaacgaAGGTAGTGT-3' |

**Tabelle 2 - Tbet-spezifische DNAzyme**

| **SEQ ID** | **Name** | **Sequenz** |
|---|---|---|
| SEQ ID No: 71 | td 1 | 5'-TGGCTTCTAggctagctacaacgaGCCCTCGTC-3' |
| SEQ ID No: 72 | td2 | 5'-GGGCTCTGAggctagctacaacgaGCCTGGCTT-3' |
| SEQ ID No: 73 | td3 | 5'-GGGACCCCAggctagctacaacgaCGGAGCCCG-3' |
| SEQ ID No: 74 | td4 | 5'-GGTGGGGGAggctagctacaacgaCCCACCGGA-3' |
| SEQ ID No: 75 | td5 | 5'-GGCGGGGGAggctagctacaacgaCCGAGGGCC-3' |
| SEQ ID No: 76 | td6 | 5'-GGGCTGGGAggctagctacaacgaGGGCAGGGA-3' |
| SEQ ID No: 77 | td7 | 5'-CGTCGAGGAggctagctacaacgaCCGCCCCTC-3' |
| SEQ ID No: 78 | td8 | 5'-GGGCTGGCAggctagctacaacgaCTTCCCGTA-3' |
| SEQ ID No: 79 | td9 | 5'-CGATGCCCAggctagctacaacgaCCGGGGCGG-3' |
| SEQ ID No: 80 | td10 | 5'-GCTCCACGAggctagctacaacgaGCCCATCCG-3' |
| SEQ ID No: 81 | td11 | 5'-CCGGCTCCAggctagctacaacgaGATGCCCAT-3' |
| SEQ ID No: 82 | td12 | 5'-TCTCCGCAAggctagctacaacgaCCGGCTCCA-3' |
| SEQ ID No: 83 | td13 | 5'-CCGTCAGCAggctagctacaacgaGTCTCCGCA-3' |
| SEQ ID No: 84 | td14 | 5'-TCCCCGGCAggctagctacaacgaCGGCTCGGT-3' |
| SEQ ID No: 85 | td15 | 5'-CCCCCGCGAggctagctacaacgaGCTCGTCCG-3' |
| SEQ ID No: 86 | td16 | 5'-GTAGGGAGAggctagctacaacgaCCCAGGCTG-3' |
| SEQ ID No: 87 | td17 | 5'-GGGCGGGCAggctagctacaacgaCAAGGCGCC-3' |
| SEQ ID No: 88 | td18 | 5'-CGGGAAGGAggctagctacaacgaTCGCCCGCG-3' |
| SEQ ID No: 89 | td19 | 5'-TAGTCCTCAggctagctacaacgaGCGGCCCCG-3' |
| SEQ ID No: 90 | td20 | 5'-TCCCCGACAggctagctacaacgaCTCCAGTCC-3' |
| SEQ ID No: 91 | td21 | 5'-TTTCCCCGAggctagctacaacgaACCTCCAGT-3' |
| SEQ ID No: 92 | td22 | 5'-TGAGCGCGAggctagctacaacgaCCTCAGTTT-3' |
| SEQ ID No: 93 | td23 | 5'-GGACCACAAggctagctacaacgaAGGTGGTTG-3' |
| SEQ ID No: 94 | td24 | 5'-CTTGGACCAggctagctacaacgaAACAGGTGG-3' |
| SEQ ID No: 95 | td25 | 5'-AAACTTGGAggctagctacaacgaCACAACAGG-3' |
| SEQ ID No: 96 | td26 | 5'-CTGATTAAAggctagctacaacgaTTGGACCAC-3' |
| SEQ ID No: 97 | td27 | 5'-TGGTGCTGAggctagctacaacgaTAAACTTGG-3' |
| SEQ ID No: 98 | td28 | 5'-TGATGATCAggctagctacaacgaCTCTGTCTG-3' |
| SEQ ID No: 99 | td29 | 5'-TGGTGATGAggctagctacaacgaCATCTCTGT-3' |
| SEQ ID No: 100 | td30 | 5'-GCTTGGTGAggctagctacaacgaGATCATCTC-3' |
| SEQ ID No: 101 | td31 | 5'-ATGGGAACAggctagctacaacgaCCGCCGTCC-3' |
| SEQ ID No: 102 | td32 | 5'-GAATGGGAAggctagctacaacgaATCCGCCGT-3' |
| SEQ ID No: 103 | td33 | 5'-TGACAGGAAggctagctacaacgaGGGAACATC-3' |
| SEQ ID No: 104 | td34 | 5'-AGTAAATGAggctagctacaacgaAGGAATGGG-3' |
| SEQ ID No: 105 | td35 | 5'-CACAGTAAAggctagctacaacgaGACAGGAAT-3' |
| SEQ ID No: 106 | td36 | 5'-GCCCGGCCAggctagctacaacgaAGTAAATGA-3' |
| SEQ ID No: 107 | td37 | 5'-CCACAAACAggctagctacaacgaCCTGTAGTG-3' |
| SEQ ID No: 108 | td38 | 5'-GTCCACAAAggctagctacaacgaATCCTGTAG-3' |
| SEQ ID No: 109 | td39 | 5'-CCACGTCCAggctagctacaacgaAAACATCCT-3' |
| SEQ ID No: 110 | td40 | 5'-CCAAGACCAggctagctacaacgaGTCCACAAA-3' |
| SEQ ID No: 111 | td41 | 5'-CCACCAAGAggctagctacaacgaCACGTCCAC-3' |
| SEQ ID No: 112 | td42 | 5'-GCTGGTCCAggctagctacaacgaCAAGACCAC-3' |
| SEQ ID No: 113 | td43 | 5'-GCTCTGGTAggctagctacaacgaCGCCAGTGG-3' |
| SEQ ID No: 114 | td44 | 5'-CTGCACCCAggctagctacaacgaTTGCCGCTC-3' |
| SEQ ID No: 115 | td45 | 5'-CACACTGCAggctagctacaacgaCCACTTGCC-3' |
| SEQ ID No: 116 | td46 | 5'-CTTTCCACAggctagctacaacgaTGCACCCAC-3' |
| SEQ ID No: 117 | td47 | 5'-GCCTTTCCAggctagctacaacgaACTGCACCC-3' |
| SEQ ID No: 118 | td48 | 5'-TTCCTGGCAggctagctacaacgaGCTGCCCTC-3' |
| SEQ ID No: 119 | td49 | 5'-GTGGACGTAggctagctacaacgaAGGCGGTTT-3' |
| SEQ ID No: 120 | td50 | 5'-CCGGGTGGAggctagctacaacgaGTACAGGCG-3' |
| SEQ ID No: 121 | td51 | 5'-CCTGGCGCAggctagctacaacgaCCAGTGCGC-3' |
| SEQ ID No: 122 | td52 | 5'-CAAATGAAAggctagctacaacgaTTCCTGGCG-3' |
| SEQ ID No: 123 | td53 | 5'-TTTCCCAAAggctagctacaacgaGAAACTTCC-3' |
| SEQ ID No: 124 | td54 | 5'-ATTGTTGGAggctagctacaacgaGCCCCCTTG-3' |
| SEQ ID No: 125 | td55 | 5'-TGGGTCACAggctagctacaacgaTGTTGGACG-3' |
| SEQ ID No: 126 | td56 | 5'-TCTGGGTCAggctagctacaacgaATTGTTGGA-3' |
| SEQ ID No: 127 | td57 | 5'-GCACAATCAggctagctacaacgaCTGGGTCAC-3' |
| SEQ ID No: 128 | td58 | 5'-GGAGCACAAggctagctacaacgaCATCTGGGT-3' |
| SEQ ID No: 129 | td59 | 5'-ACTGGAGCAggctagctacaacgaAATCATCTG-3' |
| SEQ ID No: 130 | td60 | 5'-ATGGAGGGAggctagctacaacgaTGGAGCACA-3' |
| SEQ ID No: 131 | td61 | 5'-TGGTACTTAggctagctacaacgaGGAGGGACT-3' |
| SEQ ID No: 132 | td62 | 5'-GGGCTGGTAggctagctacaacgaTTATGGAGG-3' |
| SEQ ID No: 133 | td63 | 5'-TCAACGATAggctagctacaacgaGCAGCCGGG-3' |
| SEQ ID No: 134 | td64 | 5'-CCTCAACGAggctagctacaacgaATGCAGCCG-3' |
| SEQ ID No: 135 | td65 | 5'-TCACCTCAAggctagctacaacgaGATATGCAG-3' |
| SEQ ID No: 136 | td66 | 5'-CGTCGTTCAggctagctacaacgaCTCAACGAT-3' |
| SEQ ID No: 137 | td67 | 5'-GTAAAGATAggctagctacaacgaGCGTGTTGG-3' |
| SEQ ID No: 138 | td68 | 5'-AAGTAAAGAggctagctacaacgaATGCGTGTT-3' |
| SEQ ID No: 139 | td69 | 5'-GGCAATGAAggctagctacaacgaTGGGTTTCT-3' |
| SEQ ID No: 140 | td70 | 5'-TCACGGCAAggctagctacaacgaGAACTGGGT-3' |
| SEQ ID No: 141 | td71 | 5'-AGGCAGTCAggctagctacaacgaGGCAATGAA-3' |
| SEQ ID No: 142 | td72 | 5'-ATCTCGGCAggctagctacaacgaTCTGGTAGG-3' |
| SEQ ID No: 143 | td73 | 5'-GCTGAGTAAggctagctacaacgaCTCGGCATT-3' |
| SEQ ID No: 144 | td74 | 5'-TATTATCAAggctagctacaacgaTTTCAGCTG-3' |
| SEQ ID No: 145 | td75 | 5'-GGGTTATTAggctagctacaacgaCAATTTTCA-3' |
| SEQ ID No: 146 | td76 | 5'-AAGGGGTTAggctagctacaacgaTATCAATTT-3' |
| SEQ ID No: 147 | td77 | 5'-CTCCCGGAAggctagctacaacgaCCTTTGGCA-3' |
| SEQ ID No: 148 | td78 | 5'-GTACATGGAggctagctacaacgaTCAAAGTTC-3' |

**Tabelle 3 - Beispiel einer erfindungsgemäßen Zusammensetzung**

| **Substanz** | **[% w/w]** |
|---|---|
| DNAzym | 0,01-0,75 |
| Nuklease-Inhibitor | *variabel* |
| *Beispiel*: DNase-Inhibitor | |

**Tabelle 4 - Mögliche zusätzliche Komponenten der Zusammensetzung aus Tabelle 3**

| **Substanz** | **[% w/w]** |
|---|---|
| Salz und/oder Kation | *variabel* |
| *Beispiel*: Na, Mg, K, Li, Ca, Fe, Cu, Ag, HPO₄²⁻, H₂PO₄⁻, | |
| EDTA | *variabel* |
| TRIS | *variabel* |
| Lösungsvermittler | *variabel* |
| *Beispiel*: Glycerolderivate, Polyethylenglycole, Lecithine | |
| Konservierungsstoff | *variabel* |
| *Beispiel*: Paraben | |

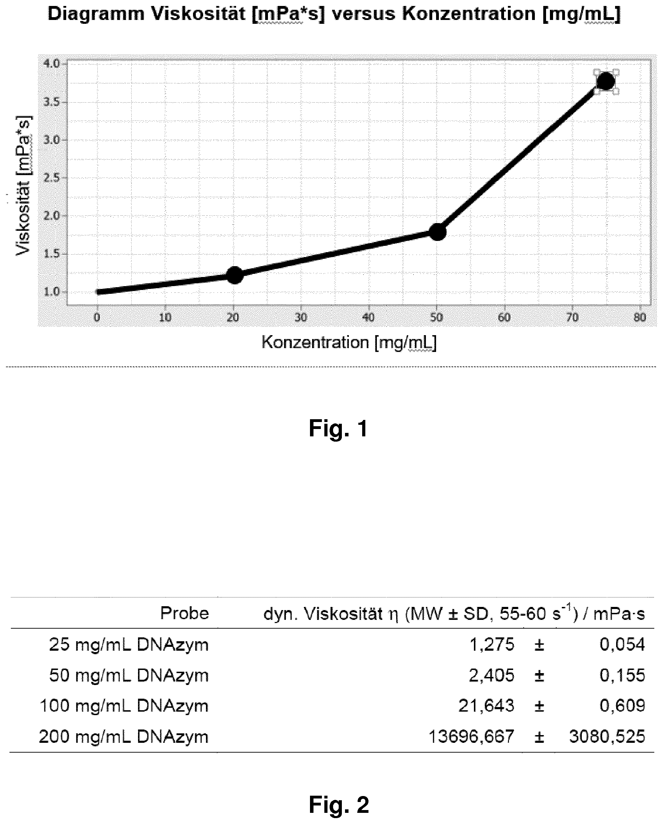

In Figur 1 ist eine grafische Darstellung der Viskosität einer DNAzym-haltigen Zusammensetzung in Abhängigkeit ihrer Konzentration gezeigt. Das DNAzym kann dabei die Expression von GATA-3 spezifisch herunterregulieren und/oder die Expression von Tbet spezifisch herunterregulieren.
Nach bevorzugter Ausführung sind vor allem DNAzyme aus einer Gruppe umfassend die DNAzyme hdg1 bis hdg70 oder aus einer Gruppe umfassend die DNAzyme td1 bis td78 vorgesehen. Insbesondere soll das DNAzym hgd40 (SEQ ID No. 40) eingesetzt werden, das die Sequenz GTGGATGGAggctagctacaacgaGTCTTGGAG aufweist und das die Expression von GATA-3 spezifisch inhibiert.

Der Graph aus Figur 1 zeigt die Viskosität einer hgd40-haltigen Zusammensetzung. Entlang der Y-Achse des Graphen aus Figur 1 wird die Viskosität der Zusammensetzung in mPa·s aufgetragen, die X-Achse gibt die DNAzym-Konzentration der Zusammensetzung an. Aus der Darstellung ergibt sich, dass die Viskosität der Zusammensetzung im Wesentlichen exponentiell zunimmt. Dabei ist insbesondere ab einer Konzentration von 50 mg/ml und höher ein starker Anstieg der Viskosität zu beobachten. Bereits bei einer Konzentration von etwa 75 mg/ml ist eine Viskosität von 3,5 mPa·s zu verzeichnen.

In Figur 2 sind Viskositätsmessergebnisse einer DNAzym-haltigen Zusammensetzung in Abhängigkeit ihrer Konzentration gezeigt. Sie verdeutlichen denselben Zusammenhang von Konzentration und Viskosität wie die Grafik aus Figur 2. So steigt die Viskosität bei einer DNAzym-Konzentration zwischen 50 mg/ml und 100 mg/ml exponentiell an. Diese Korrelation ist für andere DNA-Moleküle in der Regel nicht derart ausgeprägt zu beobachten; üblicherweise steigt die Viskosität einer DNAhaltigen Lösung im Wesentlichen linear mit steigender DNA-Konzentration.

Bei einer Viskosität von über 3,5 mPa·s ist der Druck, den ein gängiges Inhalationsgerät zum Zerstäuben der Zusammensetzung ausüben müsste, zu hoch. Das kann dazu führen, dass die Inhalatoren, die üblicherweise für die Behandlung entsprechender Patienten verwendet werden, bereits nach kurzer verstopfen oder verschmieren. Dieses Risiko wird durch eine Zusammensetzung, deren DNAzym-Konzentration niedriger ist als 75 mg/ml, signifikant minimiert.

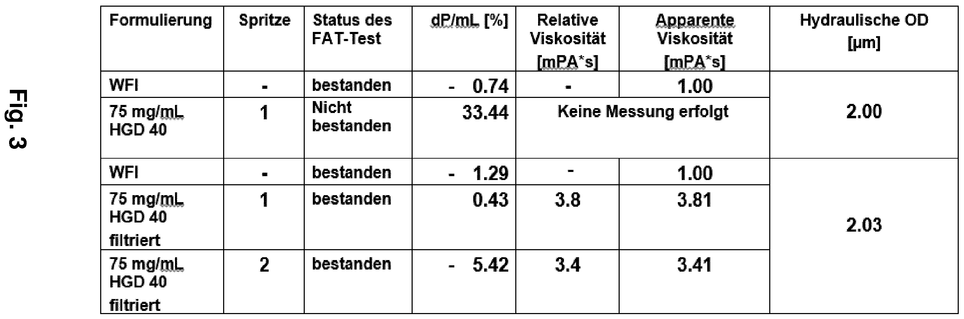

In Figur 3 sind Messergebnisse eines FATs von Zusammensetzungen mit unterschiedlichen DNAzym-Konzentrationen in tabellarischer Form dargestellt. Ein FAT ("Factory Acceptance Test") ist mit einer Werksabnahme vergleichbar, in der die Anwendbarkeit eines Produkts getestet werden soll. Die Tabelle belegt, dass eine DNAzym-Konzentration von über 75 mg/ml in der erfindungsgemäßen Zusammensetzung nicht mehr durch die Inhalationsgeräte aus dem Test zerstäubt werden kann (zweite Ergebniszeile der Tabelle aus Figur 3). Wird eine Zusammensetzung mit einer DNAzym-Konzentration von 75 mg/ml im Vorfeld des Testlaufs steril gefiltert, so wird die Konzentration auf unter 75 mg/ml herabgesenkt. Eine Zusammensetzung mit einer derartigen DNAzym-Konzentration kann mit den im Test verwendeten Zerstäubern zerstäubt werden, wie der vierten und fünften Ergebniszeile der Tabelle aus Figur 3 entnommen werden kann. Der FAT gibt die Viskosität in dP/ml an. Diese sollte zum Bestehen des hier durchgeführten FATs unter 5 % liegen. Die Abkürzung "WFI" in der ersten und dritten Zeile steht für "Water for Injection" und ist als Kontrolle zu verstehen - entsprechend korrespondieren die "WFI"-Kontrollen mit einer Viskosität von 1 dP/ml.

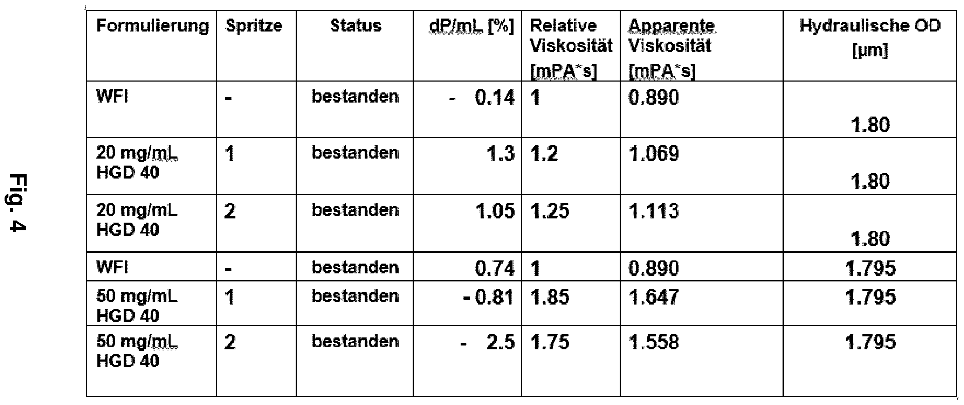

In Figur 4 sind Messergebnisse eines FATs der erfindungsgemäßen Zusammensetzung mit DNAzym-Konzentrationen von 20 mg/ml bzw. 50 mg/ml gezeigt. Die Ergebnisse belegen, dass derartige Zusammensetzungen den FAT bestehen (Status FAT Test) und sich mittels gängiger Inhalationsgeräte zerstäuben lassen ("bestanden" in den Ergebniszeilen).

Die Messergebnisse aus den Figuren 3 und 4 belegen also, dass sich die erfindungsgemäße Zusammensetzung mit einer DNAzym-Konzentrationen von unter 75 mg/ml ideal zur Behandlung eines an einer mit chronischen Entzündungen einhergehenden Atemwegserkrankung leidenden Patienten eignet, weil eine derartige Zusammensetzung zerstäubt und somit als Aerosol verwendet werden kann. Dabei werden bevorzugterweise DNAzym-Konzentrationen von 20 mg/ml bis 50 mg/ml eingesetzt.

Man erkennt mithin, dass die Erfindung eine Zusammensetzung zur Behandlung eines Patienten betrifft, der an einer mit chronischen Entzündungen einhergehenden Atemwegserkrankung leidet, wobei die Zusammensetzung mindestens ein DNAzym umfasst, das die Expression von GATA-3 spezifisch herunterreguliert und/oder mindestens ein DNAzym umfasst, das die Expression von Tbet spezifisch herunterreguliert. Die Zusammensetzung ist dadurch gekennzeichnet, dass die Konzentration des DNAzyms in der Zusammensetzung niedriger als 75 mg/ml ist, wobei die Konzentration des DNAzyms in der Zusammensetzung vorzugsweise zwischen 20 mg/ml und 50 mg/ml liegt.

Die Erfindung betrifft außerdem ein Verfahren zur Herstellung der erfindungsgemäßen Zusammensetzung, diese mindestens ein DNAzym umfasst, das die Expression von GATA-3 spezifisch herunterreguliert und/oder mindestens ein DNAzym umfasst, das die Expression von Tbet spezifisch herunterreguliert. Auch hierbei ist vorgesehen, dass die Konzentration des DNAzyms in der Zusammensetzung niedriger als 75 mg/ml ist, vorzugsweise niedriger ist als 3,5 mPa·s.

Die Verwendung der Zusammensetzung ist auf ein Arzneimittel gerichtet, das zur Behandlung eines an einer mit chronischen Entzündungen einhergehenden Atemwegserkrankung leidenden Patienten eingesetzt wird.

## Patentansprüche

1. Zusammensetzung zur Behandlung eines Patienten, der an einer mit chronischen Entzündungen einhergehenden Atemwegserkrankung leidet, wobei die Zusammensetzung mindestens ein DNAzym umfasst, das die Expression von GATA-3 spezifisch herunterreguliert und/oder mindestens ein DNAzym umfasst, das die Expression von Tbet spezifisch herunterreguliert, **dadurch gekennzeichnet, dass** die Konzentration des DNAzyms in der Zusammensetzung niedriger als 75 mg/ml ist.

2. Zusammensetzung nach Anspruch 1, **dadurch gekennzeichnet, dass** die Konzentration des DNAzyms in der Zusammensetzung zwischen 20 mg/ml und 50 mg/ml ist.

3. Zusammensetzung nach einem der Ansprüche 1 oder 2, **dadurch gekennzeichnet, dass** die Zusammensetzung eine Viskosität aufweist, die niedriger ist als 3,5 mPa·s.

4. Zusammensetzung nach einem der vorhergehenden Ansprüche, **dadurch gekennzeichnet, dass** das DNAzym ausgewählt ist aus einer Gruppe umfassend die DNAzyme hdg1 bis hdg70 (SEQ ID No. 1 bis SEQ ID No. 70) und/oder aus einer Gruppe umfassend die DNAzyme td1 bis td78 (SEQ ID No. 71 bis SEQ ID No. 148).

5. Zusammensetzung nach Anspruch 4, **dadurch gekennzeichnet, dass** das DNAzym die Sequenz hdg40 GTGGATGGAggctagctacaacgaGTCTTGGAG (SEQ ID No. 40) aufweist.

6. Zusammensetzung nach einem der vorhergehenden Ansprüche, **dadurch gekennzeichnet, dass** die Zusammensetzung mindestens einen Nuklease-Inhibitor aufweist.

7. Zusammensetzung nach Anspruch 6, **dadurch gekennzeichnet, dass** der Nuklease-Inhibitor Desoxyribonukleasen spezifisch inaktiviert.

8. Zusammensetzung nach einem der vorhergehenden Ansprüche, **dadurch gekennzeichnet, dass** die Zusammensetzung mindestens ein Salz und/oder mindestens ein Kation umfasst.

9. Zusammensetzung nach einem der vorhergehenden Ansprüche, **dadurch gekennzeichnet, dass** die Zusammensetzung mindestens einen anorganischen und/oder organischen Zusatz und/oder einen Lösungsvermittler und/oder einen Konservierungsstoff umfasst.

10. Verfahren zur Herstellung der Zusammensetzung nach einem der Ansprüche 1 bis 9, **dadurch gekennzeichnet, dass** das Verfahren zumindest die folgenden Schritte umfasst:
a) Ansetzen einer DNAzym-haltigen Lösung, wobei die DNAzym-Konzentration in der Lösung 80 mg/ml nicht übersteigt;
b) Filtration der Lösung aus Schritt a), bis die Lösung eine DNAzym-Konzentration von unter 75 mg/ml aufweist;

11. Verfahren nach Anspruch 10, **dadurch gekennzeichnet, dass** das Verfahren außerdem mindestens einen der folgenden Schritte umfasst:
• Hinzufügen des mindestens einen Nuklease-Inhibitors zu der Lösung aus Schritt b);
• Hinzufügen des mindestens einen Salzes und/oder des mindestens einen Kations zur Lösung aus Schritt b);
• Hinzufügen des mindestens einen anorganischen und/oder organischen Zusatzes zur Lösung aus Schritt b);
• Hinzufügen des Lösungsvermittlers und/oder des Konservierungsstoffs zur Lösung aus Schritt b).

12. Verwendung der Zusammensetzung nach einem der Ansprüche 1 bis 9 als Arzneimittel zur Behandlung eines an einer mit chronischen Entzündungen einhergehenden Atemwegserkrankung leidenden Patienten.

13. Verwendung nach Anspruch 12, **dadurch gekennzeichnet, dass** die Zusammensetzung in Form eines Aerosols appliziert wird.
